# EUROPEAN PATENT SPECIFICATION

(11) **EP 3 807 642 B1**
(45) Date of publication and mention of the grant of the patent: **12.06.2024**
(21) Application number: 19799471.8
(22) Date of filing: 10.05.2019
(51) Int. Cl.: G01N 33/566

(54) **METHODS FOR IDENTIFYING ANTIGEN-SPECIFIC T CELL RECEPTORS**
VERFAHREN ZUR IDENTIFIZIERUNG VON ANTIGEN-SPEZIFISCHEN T-ZELLREZEPTOREN
MÉTHODES D'IDENTIFICATION DE RÉCEPTEURS DE LYMPHOCYTES T SPÉCIFIQUES D'UN ANTIGÈNE

(30) Priority: 11.05.2018 US 201862670407 P; 21.06.2018 US 201862688084 P
(43) Date of publication of application: 21.04.2021
(73) Proprietor: Memorial Sloan-Kettering Cancer Center, New York, NY 10065 (US)
(72) Inventor: KLEBANOFF, Christopher A., New York, NY 10022 (US); CHANDRAN, Smita S., Long Island City, NY 11101 (US)
(74) Representative: Zacco Denmark A/S
(86) International application number: PCT/US2019/031743
(87) International publication number: WO 2019/217831

(56) References cited:
- EP-A1- 3 173 474
- WO-A1-2016/146618
- WO-A1-2018/057447
- WO-A2-2008/059252
- WO-A2-2008/059252
- WO-A2-2014/160030
- WO-A2-2015/071763
- WO-A2-2017/120428
- US-A1- 2004 209 363
- US-A1- 2014 212 446
- SHAO HONGWEI ET AL: "Identification of peptide-specific TCR genes byin vitropeptide stimulation and CDR3 length polymorphism analysis", CANCER LETTERS, NEW YORK, NY, US, vol. 363, no. 1, 15 April 2015 (2015-04-15), pages 83-91, XP029224476, ISSN: 0304-3835, DOI: 10.1016/J.CANLET.2015.04.001
- FANG QIONG ET AL: "Cartilage-reactive T cells in rheumatoid synovium", INTERNATIONAL IMMUNOLOGY, vol. 12, no. 5, 1 May 2000 (2000-05-01), pages 659-669, XP055900953, GB ISSN: 0953-8178, DOI: 10.1093/intimm/12.5.659 Retrieved from the Internet: URL:https://watermark.silverchair.com/659. pdf?token=AQECAHi208BE49Ooan9kkhW_Ercy7Dm3 ZL_9Cf3qfKAc485ysgAAAtkwggLVBgkqhkiG9w0BBw agggLGMIICwgIBADCCArsGCSqGSIb3DQEHATAeBglg hkgBZQMEAS4wEQQMsrIN1dK11lEIkuFHAgEQgIICjK SPe0qtvlm3XT-9clq6T0AYPj5sS6TvM3CFGWloj5UX vnRPk1Y4tVv7gQuy-POt8sHnWrJcX8ytZMcO7jMNIZ QWyvKScnsZ>
- KAZUE ARIMOTO-MIYAMOTO ET AL: "Optimal stimulation for CD70 induction on human monocyte-derived dendritic cells and the importance of CD70 in naive CD4+ T-cell differentiation", IMMUNOLOGY, WILEY-BLACKWELL PUBLISHING LTD, GB, vol. 130, no. 1, 26 February 2010 (2010-02-26), pages 137-149, XP071275698, ISSN: 0019-2805, DOI: 10.1111/J.1365-2567.2010.03220.X
- GILBOA ELI: "Knocking the SOCS1 off dendritic cells", NATURE BIOTECHNOLOGY, NATURE PUBLISHING GROUP US, NEW YORK, vol. 22, no. 12, 1 December 2004 (2004-12-01), pages 1521-1522, XP037159608, ISSN: 1087-0156, DOI: 10.1038/NBT1204-1521

## Description

### CROSS-REFERENCE TO RELATED APPLICATION

This application claims priority to U.S. Provisional Application No.: 62/670,407 filed on May 11, 2018, and to U.S. Provisional Application No.: 62/688,084 filed on June 21, 2018.

### INTRODUCTION

The presently disclosed subject matter provides *in vitro* methods for identifying T cell receptors (TCRs) that target specific antigens. The presently disclosed subject matter further provides TCRs identified by the methods and immunoresponsive cells comprising such TCRs.

### BACKGROUND OF THE INVENTION

Cell-based immunotherapy is a therapy with curative potential for the treatment of cancer. T cells and other immune cells may be modified to target tumor antigens through the introduction of genetic material coding for TCRs specific to selected antigens. Targeted T cell therapy using specific TCRs has shown recent clinical success in treating hematologic malignancies. Accordingly, there are needs for novel therapeutic strategies to identify and generate TCRs targeting specific tumor antigens, and for strategies capable of inducing potent cancer eradication with minimal toxicity and immunogenicity.

WO 2015/071763 discloses cytotoxic T lymphocytes, peptide epitopes, high-throughput methods for their identification, and their uses. The reference further discloses methods and systems for identifying antigen-specific CTLs.

Shao et al. (2015) discloses that TCR genemodified peripheral blood mononuclear cells (PBMCs) could be effectively activated by mutant peptide-loaded T2 cells.

WO 2016/146618 discloses a method for preparing a nucleic acid encoding the TCR alpha chain construct (TRA) and TCR beta chain construct (TRB) of a TCR construct specific for an epitope from an antigen presented on major histocompatibility complex (MHC), comprising contacting T cells isolated from a donor with a library of antigen presenting cells (APC) comprising cells expressing all MHC I or MHC II alleles present in the donor.

Fang et al. (2000) investigates the nature of the antigens driving the T cell response, synovial tissue was obtained from a patient with chronic RA and T cells were enriched. TCR alpha-beta pairs which appeared to respond to antigen(s) in the cartilage extract were identified.

WO 2008/069252 discloses a method for isolating nucleic acids encoding a T cell receptor which recognizes a 5T4 antigen.

### SUMMARY OF THE INVENTION

The present invention is set out in the appended set of claims. In particular, the present invention provides an *in vitro* method for identifying a T cell receptor (TCR) that targets an antigen, comprising:
a) contacting a plurality of lymphocytes with a plurality of antigen presenting cells (APCs), wherein the APC comprises an antigen and at least one exogenous costimulatory ligand;
b) identifying a lymphocyte that is stimulated by the APC (APC-stimulated lymphocyte); and
c) identifying a TCR comprised by the APC-stimulated lymphocyte identified in b); wherein the lymphocytes and the APCs are from a population of peripheral blood mononuclear cells of a single donor, and optionally wherein
   (i) the APC is a mature APC,
   (ii) the APC is a monocyte-derived dendritic cell; and/or
   (iii) the lymphocyte is a T cell.

In certain embodiments, the antigen is a tumor antigen or a pathogen antigen. In certain embodiments, the antigen is selected from a group of targets, including but not limited to, tumor associated antigens, cancer germline antigens, and mutated antigens or neoantigens. In certain embodiments, the tumor associated antigens are selected from the group consisting of MART-1, tyrosinase, CEA, and MUC-1. In certain embodiments, the cancer germline antigens are selected from the group consisting of MAGE, and NY-ESO. In certain embodiments, the mutated antigens or neoantigens are selected from the group consisting of PIK3KC, BRAF, and KRAS. In certain embodiments, the antigen is selected from the group consisting of PIK3CA, BRAF, KRAS, MART-1, tyrosinase, CEA, MUC-1, MAGE, and NY-ESO. In certain embodiments, the antigen is a small peptide derived from the tumor antigen. In certain embodiments, the antigen is a small peptide that comprises fewer than 24 amino acid residues. In certain embodiments, the antigen is the full length sequence. In certain embodiments, the antigen comprises a mutation.

In certain embodiments, the APC further comprises at least one costimulatory ligand. In certain embodiments, the costimulatory ligand is selected from the group consisting of 4-1BBL, OX40L, CD40L, ICOSL, CD70 and combinations thereof. In certain embodiments, the APC comprises two costimulatory ligands comprising 4-1BBL and OX40L.

In certain embodiments, the antigen and the at least one (e.g., two, three or four) costimulatory ligands are transfected into the APC. In certain embodiments, the antigen and the costimulatory ligand are expressed through a single vector.

In certain embodiments, identifying the APC-stimulated lymphocyte comprises detecting an increase of expression of a cytokine of the APC-stimulated lymphocyte as compared to expression of the cytokine of a control lymphocyte. In certain embodiments, the control lymphocyte is a lymphocyte that has not been contacted with an APC or a lymphocyte that has been contacted with an APC that does not comprise the antigen. In certain embodiments, the cytokine is selected from the group consisting of IL-2, IFNγ, TNFα, MIP1α, CD107a, CD107b, granzyme B, perforin, IL-4 and TGF-β and combinations thereof. In certain embodiments, the method further comprises measuring the cytokine expression of the APC-stimulated lymphocyte and measuring the cytokine expression of the control lymphocyte. In certain embodiments, measuring the cytokine expression comprises polymerase chain reaction (PCR). In certain embodiments, the PCR is a quantitative PCR (qPCR).

In certain embodiments, identifying the TCR comprises obtaining a sequence of the TCR. In certain embodiments, the sequence comprises mRNA sequence, cDNA sequence, genomic DNA sequence, or a combination thereof. In certain embodiments, the obtaining the sequence of the TCR comprises nucleic acid sequencing.

In certain embodiments, the method further comprises validating the binding specificity of the TCR to the antigen. In certain embodiments, validating the binding specificity of the TCR to the antigen comprises:
i) expressing the TCR in a lymphocyte,
ii) contacting the lymphocyte of step i) with an APC comprising the antigen, and
iii) validating the binding specificity of the TCR to the antigen when the lymphocyte of step ii) is stimulated by the APC.

In certain embodiments, the stimulation of the lymphocyte by the APC is identified by detecting an increase of expression of a cytokine of the lymphocyte as compared to expression of the cytokine of a control lymphocyte. In certain embodiments, the control lymphocyte is a lymphocyte that expresses the TCR and has not been contacted with an APC or a lymphocyte that expresses the TCR that has been contacted with an APC that does not comprise the antigen. In certain embodiments, the cytokine is selected from the group consisting of IL-2, IFNγ, TNFα, MIP1α, CD107a, CD107b, Granzyme B, Perforin, IL-4 and TGF-β and combinations thereof. ,

In certain embodiments, the lymphocyte is a T cell.

Disclosed herein is a T cell receptor (TCR) identified by any method disclosed herein. The TCR comprises an α chain and a β chain. The TCR may comprise a γ chain and a δ chain. The TCR may be recombinantly expressed, or expressed from a vector. The vector may be a γ-retroviral vector.

Disclosed is further an isolated immunoresponsive cell comprising any TCR. The immunoresponsive cell may be transduced with the TCR. The TCR may be constitutively expressed on the surface of the immunoresponsive cell. The immunoresponsive cell may be selected from the group consisting of a T cell, a Natural Killer (NK) cell, a human embryonic stem cell, a lymphoid progenitor cell, a T cell-precursor cell, and a pluripotent stem cell from which lymphoid cells may be differentiated. The T cell may be selected from the group consisting of a cytotoxic T lymphocyte (CTL), a regulatory T cell, and central memory T cells.

The Disclosed is further a composition comprising any immunoresponsive cell disclosed herein. The composition may be a pharmaceutical composition comprising a pharmaceutically acceptable carrier.

Disclosed herein is further an *in vitro* method for producing an immunoresponsive cell that binds to an antigen of interest, comprising introducing into the immunoresponsive cell a nucleic acid sequence that encodes any TCR disclosed herein. The immunoresponsive cell may be useful for treating or preventing a malignancy in a subject,such as breast cancer, ovarian cancer, bladder cancer, pancreatic cancer or other solid malignancies.

Disclosed is further a kit for treating or preventing a malignancy, comprising any immunoresponsive cell disclosed herein, any isolated nucleic acid molecule disclosed herein or any vector disclosed herein, optionally the kit further comprises written instructions for using the immunoresponsive cell for treating a subject having a malignancysuch as breast cancer, ovarian cancer, bladder cancer, pancreatic cancer or other solid malignancies.

The antigen presenting cell (APC) used by the method of the present invention comprises at least one costimulatory ligand. In certain embodiments, the costimulatory ligand is selected from the group consisting of 4-1BBL, OX40L, CD40L, ICOSL, CD70, and combinations thereof. In certain embodiments, the APCs comprise two costimulatory ligands comprising 4-1BBL and OX40L. In certain embodiments, the antigen and the costimulatory ligand are transfected into the APC. In certain embodiments, the antigen and the costimulatory ligand are expressed through a single vector. In certain embodiments, the APC is a professional APC selected from the group consisting of dendritic cells, macrophages and B cells. In certain embodiments, the APC is a monocyte-derived dendritic cell. In certain embodiments, the APC is a matured APC. In certain embodiments, the mature APC is obtained by treating an APC with LPS and/or IFNγ.

Disclosed herein is further the preparation of cells comprising any APC disclosed herein and a lymphocyte, e.g., a T cell.

### BRIEF DESCRIPTION OF THE FIGURES

The following Detailed Description, given by way of example but not intended to limit the invention to specific embodiments described, may be understood in conjunction with the accompanying drawings.

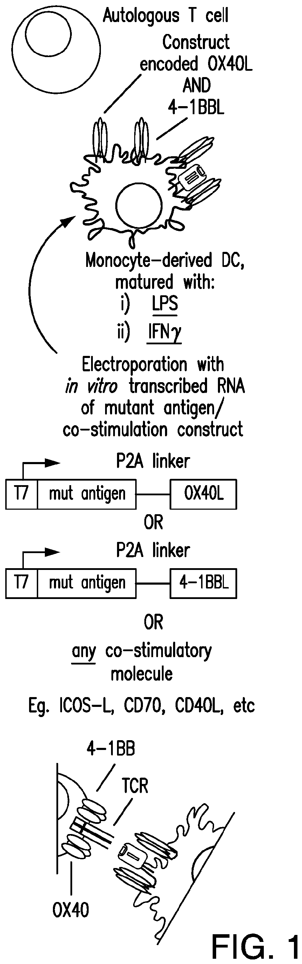

Figure 1 depicts the general mechanism of the interaction between T cells and antigen presenting cells (APCs). Constructs expressing an antigen of interest can be transfected to APCs to stimulate a T cell. Costimulatory ligands, *e*.*g*., 4-1BBL, OX40L, ICOSL, CD40L and CD70 can be expressed in the APCs to enhance T cells stimulation.

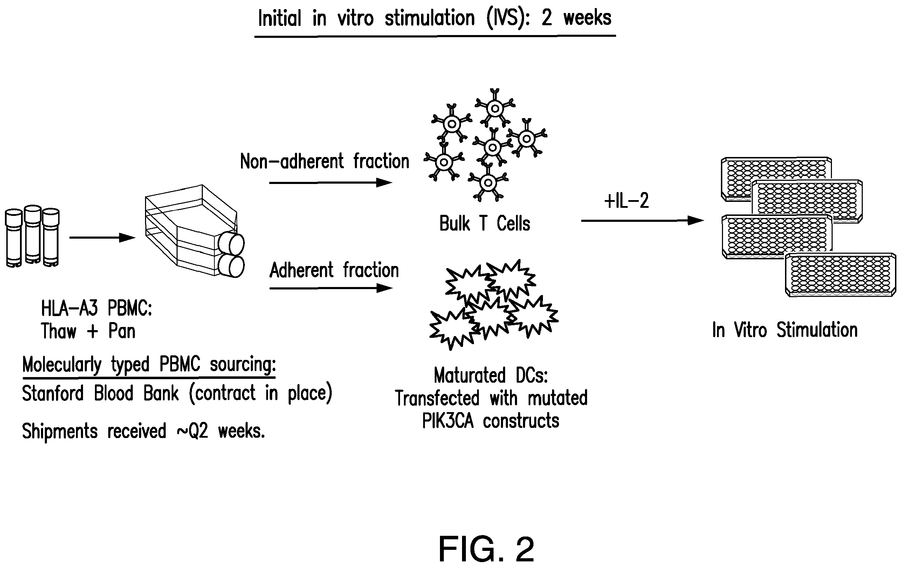

Figure 2 depicts *in vitro* sensitization (IVS) of donor-derived cells. PBMCs from healthy donors stimulated with autologous mature antigen-presenting cells were transfected with mRNA encoding a 65 amino acid segment of the PIK3CA gene flanking common hotspot mutations. Cells received *in vitro* antigen stimulation two/three times over a period of two/three weeks in the presence of low-dose IL-2 (90 IU/mL).

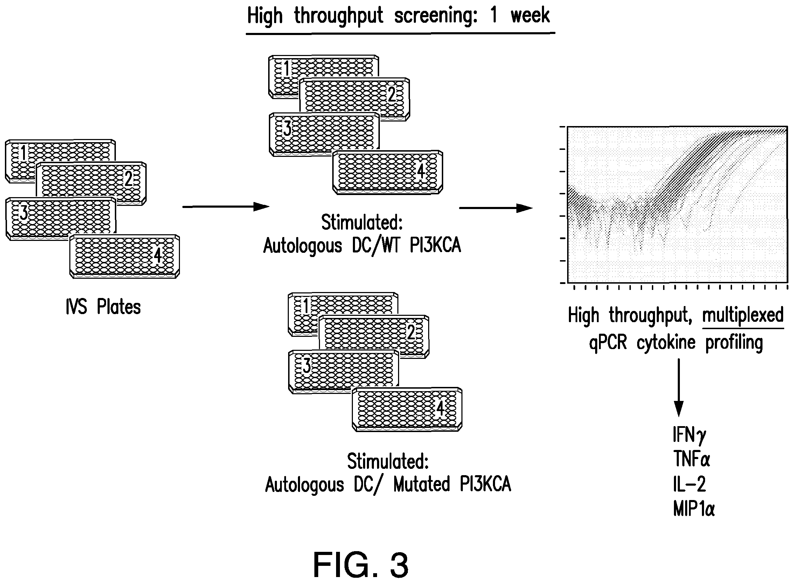

Figure 3 depicts high throughput reactivity screen. Individual *in vitro* sensitized microwells were tested for mutation-specific reactivity by incubating with autologous APCs transfected with mRNA encoding either the wild-type (WT) or mutated PIK3CA. Mutation-specific recognition was determined by the preferential upregulation of mRNA transcript of acute inflammatory markers such as IFN-g and TNF-a by high throughput quantitative PCR.

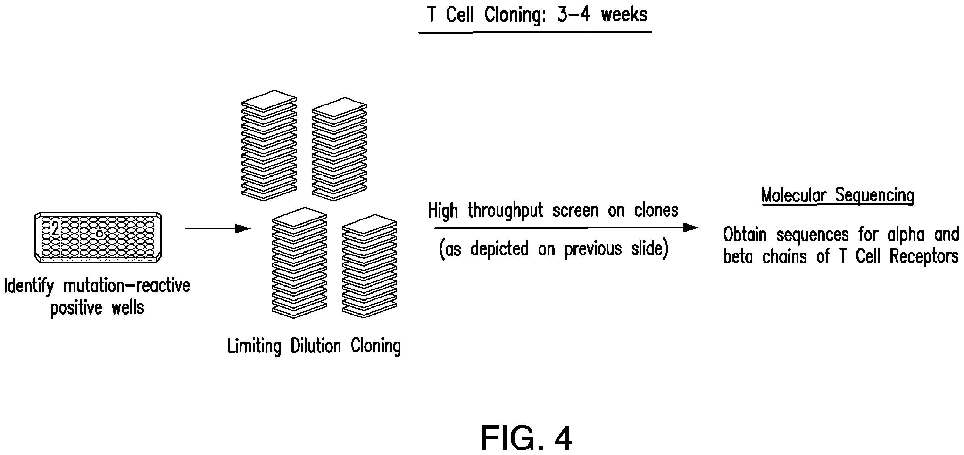

Figure 4 depicts isolation of mutation-specific T cells. Positive microwells underwent limiting dilution cloning to derive T cell clones of a single specificity. Individual clones were screened for mutation-specific recognition using previously described methodology; molecular sequencing was performed on positive clones to derive the alpha and beta chain sequences of its T cell receptor.

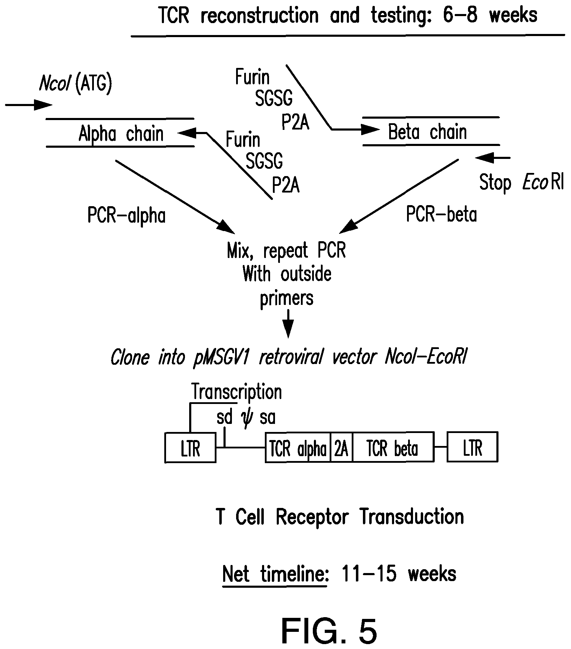

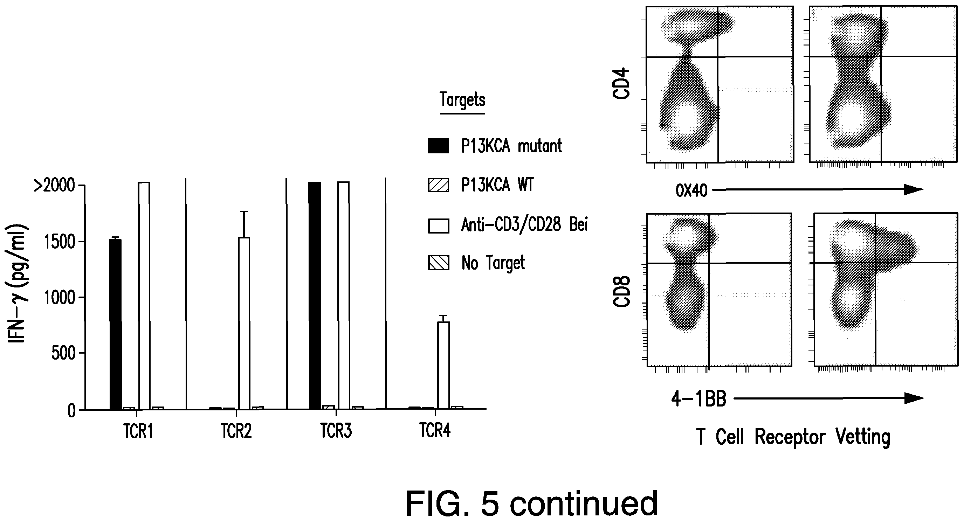

Figure 5 depicts TCR reconstruction and conference of reactivity. The genetic sequences encoding the alpha and beta chains of the mutation-specific TCR were cloned into a retroviral vector and stably integrated into allogeneic PBMC. Conference of reactivity to mutation-specific transfectants and HLA-matched tumor cell line targets expressing the mutant antigen were determined by IFN-g secretion and upregulation of costimulatory molecules such as 4-1BB and OX40. A representative schema of this process is shown as an example.

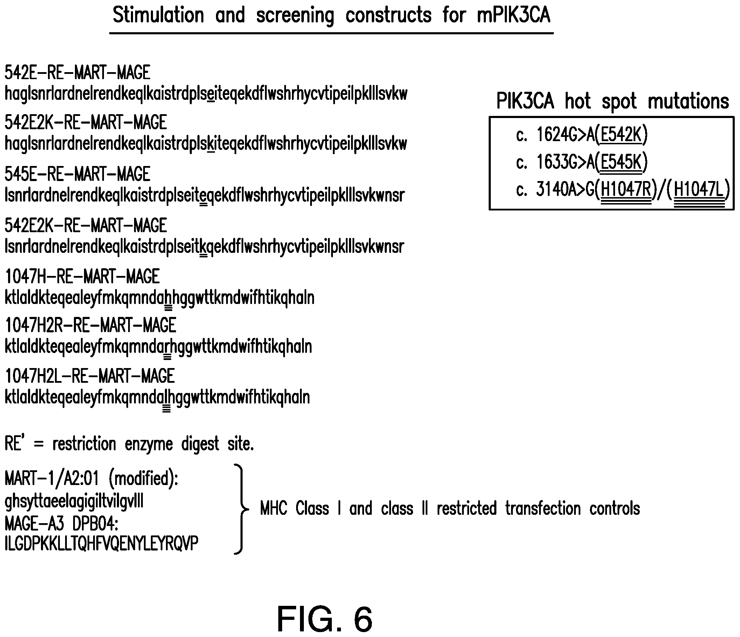

Figure 6 depicts stimulation and screening constructs for mutant PIK3CA.

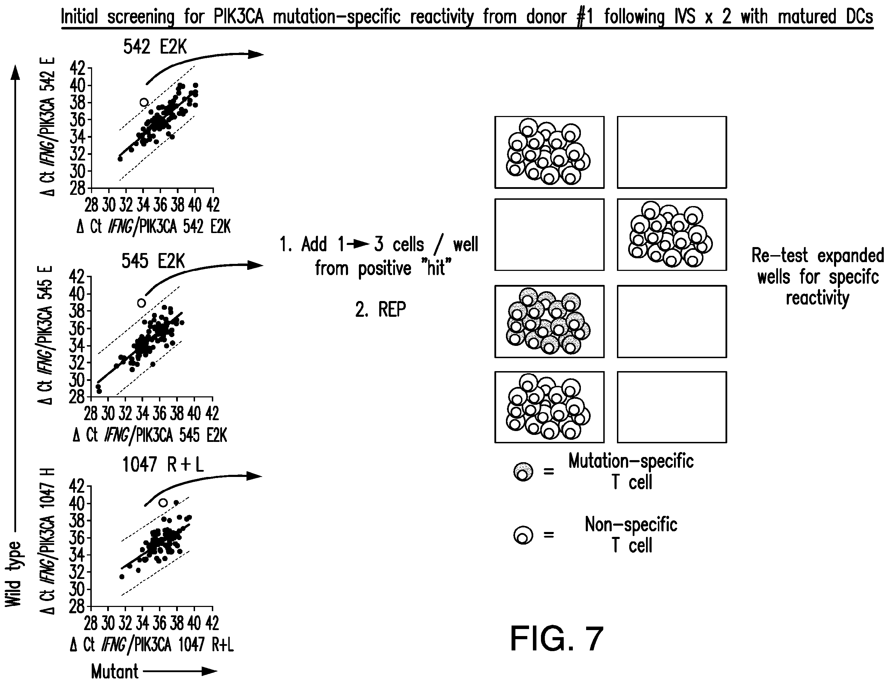

Figure 7 depicts identification of mutation-reactive microwells following *in vitro* sensitization. Delta CT values from sister wells against mutated or WT PI3KCA are plotted on the X and Y axis respectively. Each dot represents an individual sensitized microwell. Mutation-specific recognition of the hotspot mutations, E542K in the top panel, E545K mutation in the middle panel and the H1047R/L in the bottom panel are shown. Dots indicated by the arrows representing wells that preferentially upregulate IFN-g transcript in response to mutated PIK3CA were selected to undergo limiting dilution cloning to derive mutation-specific T cell clones.

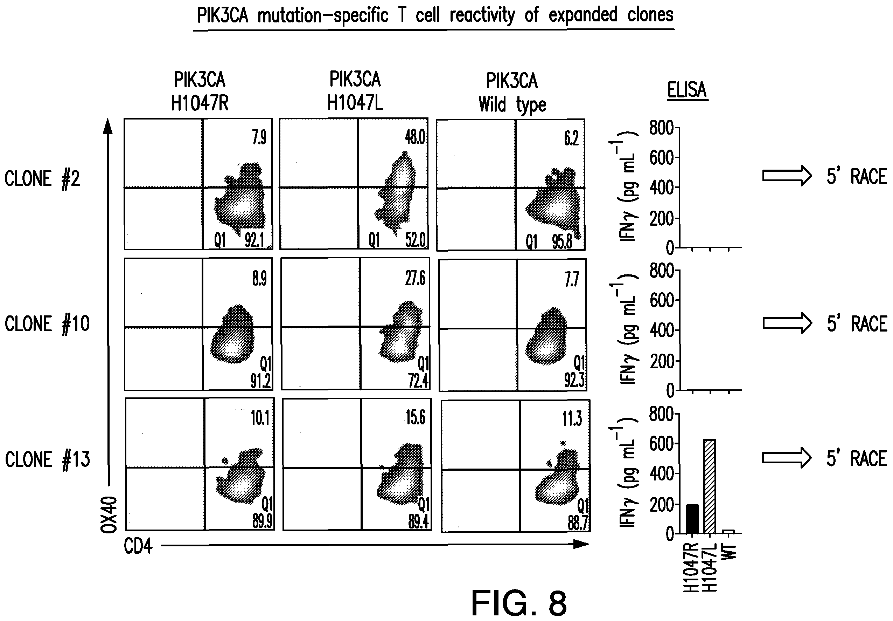

Figure 8 depicts screening of clones derived from limiting dilution. Growth-positive wells following limiting dilution cloning were screened for PIK3CA mutation-specific recognition by measuring the preferential upregulation of the costimulatory molecule, OX40 (left panel) and/or the release of IFN-g (right panel) in response to the mutated antigen. Positive clones were selected to undergo molecular sequencing to derive the genetic code for the alpha and beta chains of their T cell receptor.

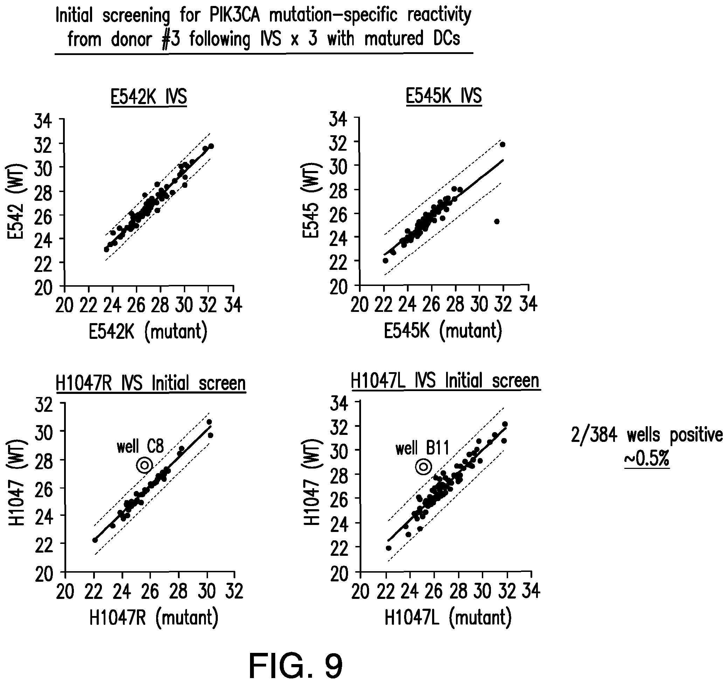

Figure 9 depicts high throughput screen and identification of PI3KCA-mutation reactive wells of Donor 3 T cells. Delta CT values from paired microwells against mutant or WT PIK3CA are plotted on the X and Y axis, respectively. Each dot represents an individual sensitized microwell (n=384) sensitized against one of four PI3KCA hotspot mutations listed. Well C8 (bottom left) derived from a H1047R-sensitized IVS and Well B11 (bottom right) derived from a H1047L-sensitized IVS were mutation-specific, as determined by preferential upregulation of IFN-g mRNA to mutant antigen.

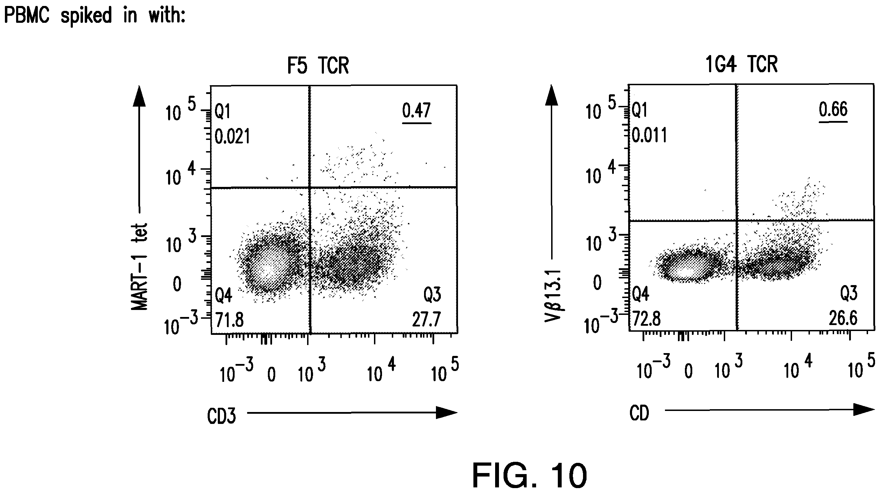

Figure 10 depicts validation of single-cell sequencing platform to correctly retrieve and quantify paired TCRa/b gene sequences from bulk populations.

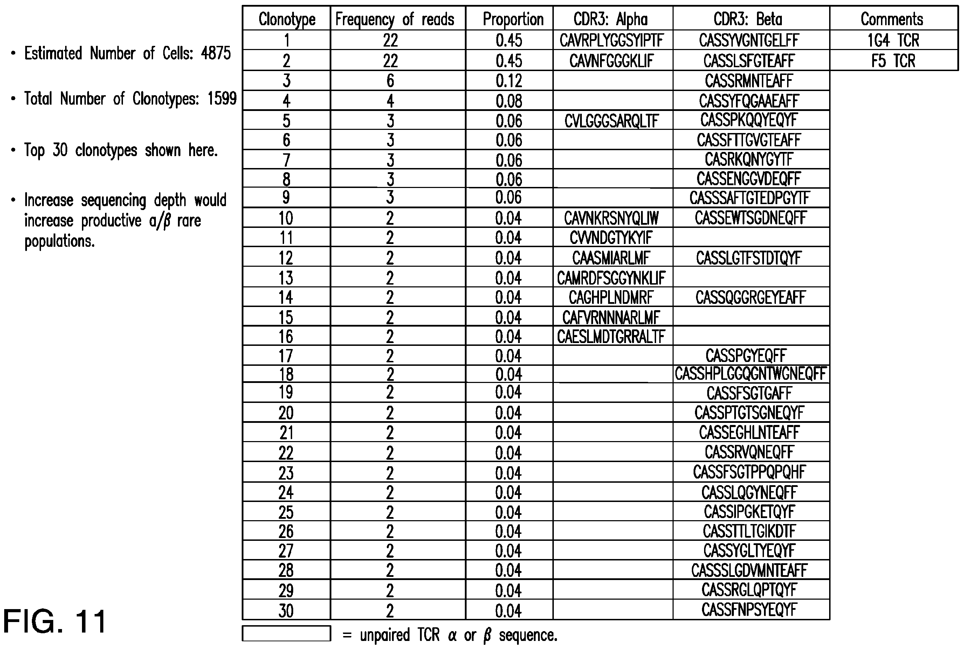

Figure 11 depicts that sequencing correctly identified, paired, and quantified known TCRs within a bulk PBMC population.

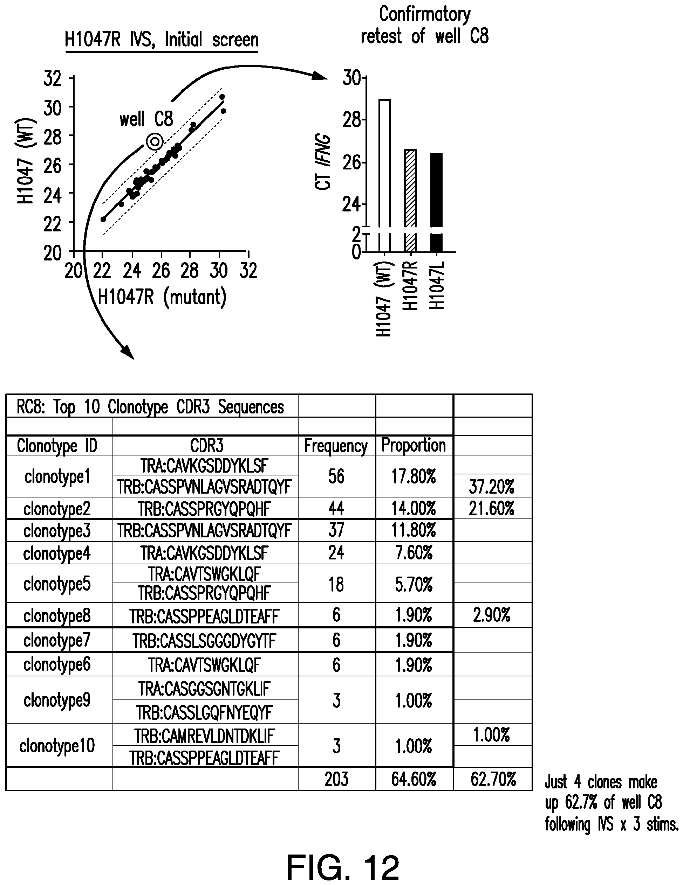

Figure 12 depicts confirmation of RC8 reactivity. Autologous APCs transfected with RNA encoding either WT or the R/ L substitutions at position 1047 in the PIK3CA gene were incubated with T cells from Well C8 (referred to as RC8). Delta CT values determined by upregulation of IFN-g transcript indicate mutation-specific recognition of both R and L hotspot mutations and no WT recognition. The table in the lower panel lists the CDR3 sequences and frequencies of the top 10 clonotypes in RC8 derived by the platform.

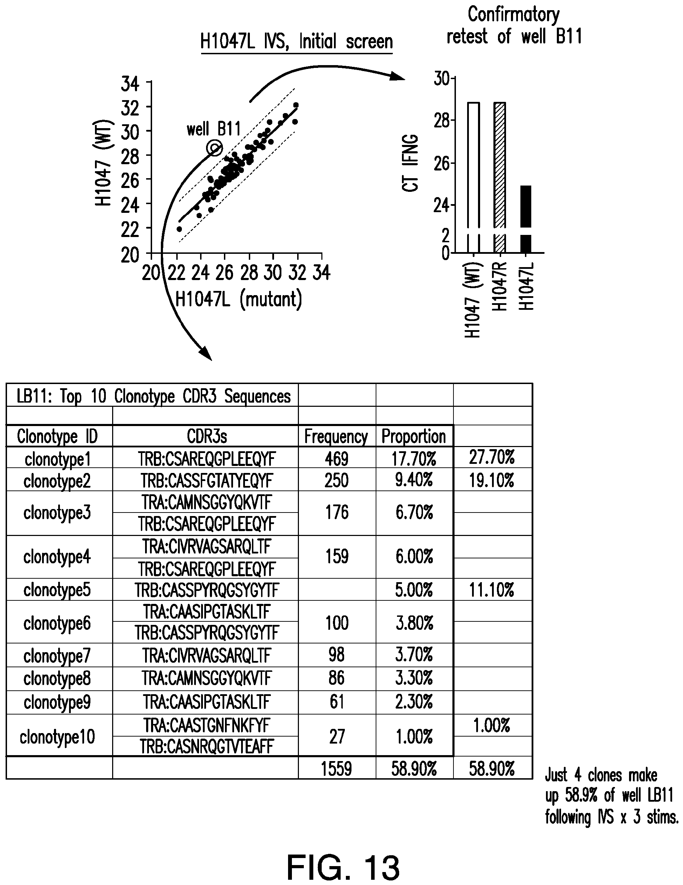

Figure 13 depicts confirmation of LB11 reactivity: Autologous APCs transfected with RNA encoding either WT or the R/L substitutions at position 1047 in the PIK3CA gene were incubated with T cells from Well B11 (referred to as LB11). Delta CT values determined by upregulation of IFN-g transcript indicate specific recognition of H1047L alone and no H1047R or WT recognition. The table in the lower panel lists the CDR3 sequences and frequencies of the top 10 clonotypes in LB11 derived by the platform.

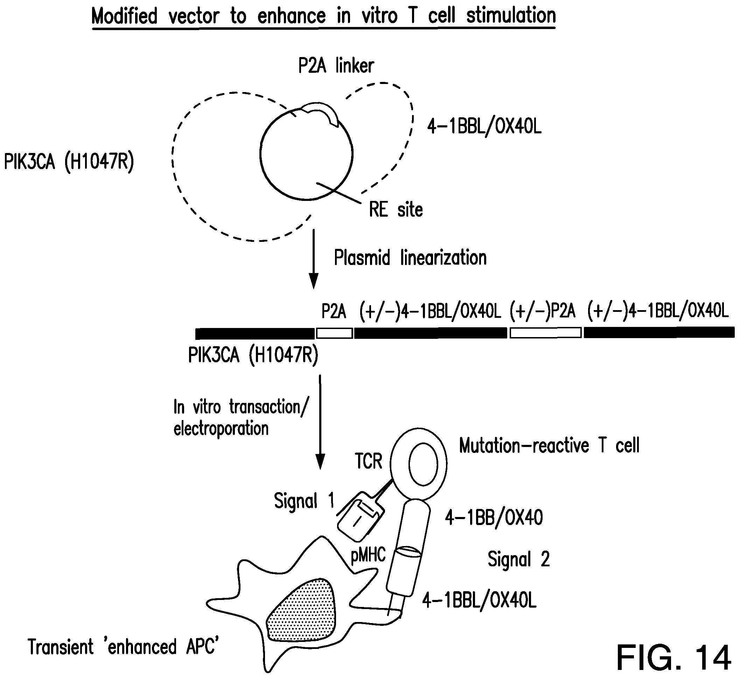

Figure 14 depicts modified vector to enhance *in vitro* T cell stimulation, where one or more costimulatory ligands are expressed in the antigen presenting cells (APCs).

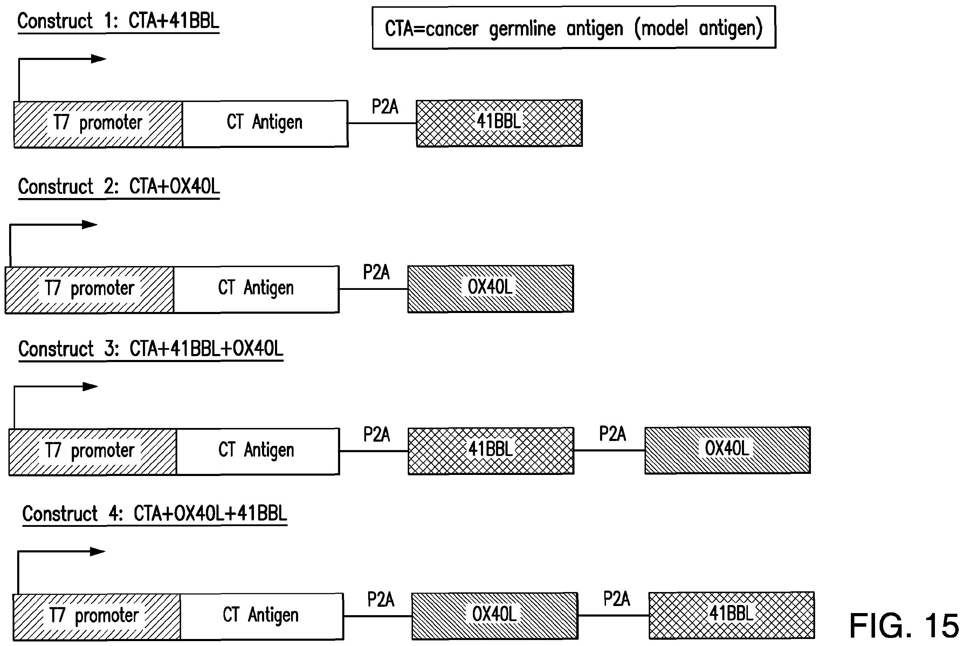

Figure 15 depicts exemplary constructs for co-expressing an antigen and one or more costimulatory ligand.

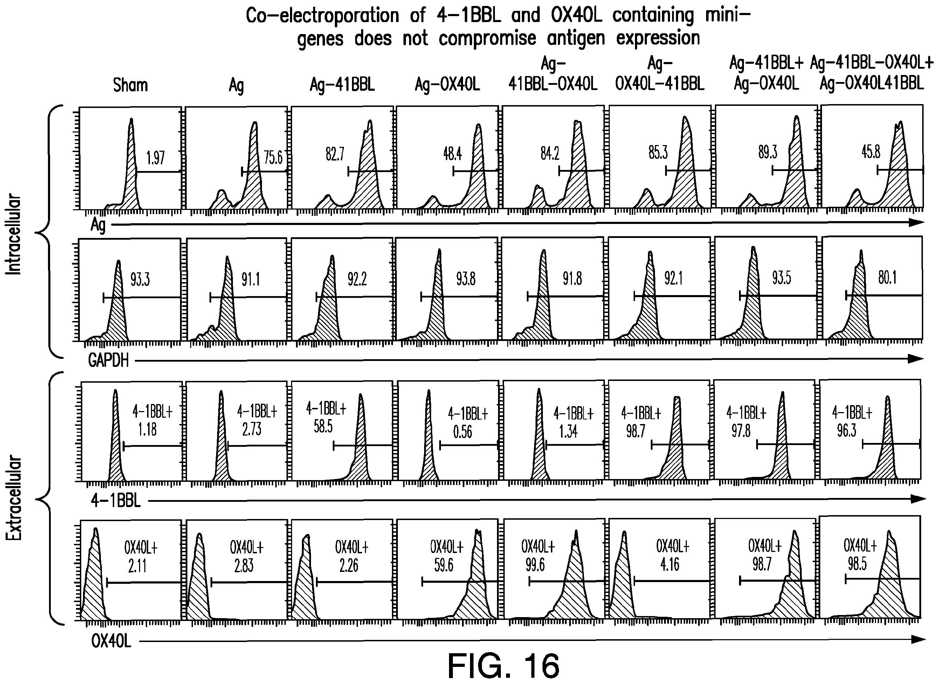

Figure 16 depicts surface expression of costimulatory ligands. Monocyte derived dendritic cells were transfected with mRNA encoding a tumor antigen of interest and the costimulatory ligand 4-1BBL or OX40L expressed alone or in tandem. Histograms depict the frequency of expression of 41BBL (top) and OX40L (bottom) at 24 hours post-transfection. Costimulatory molecules were expressed on the surface after transient transfection with the modified vector

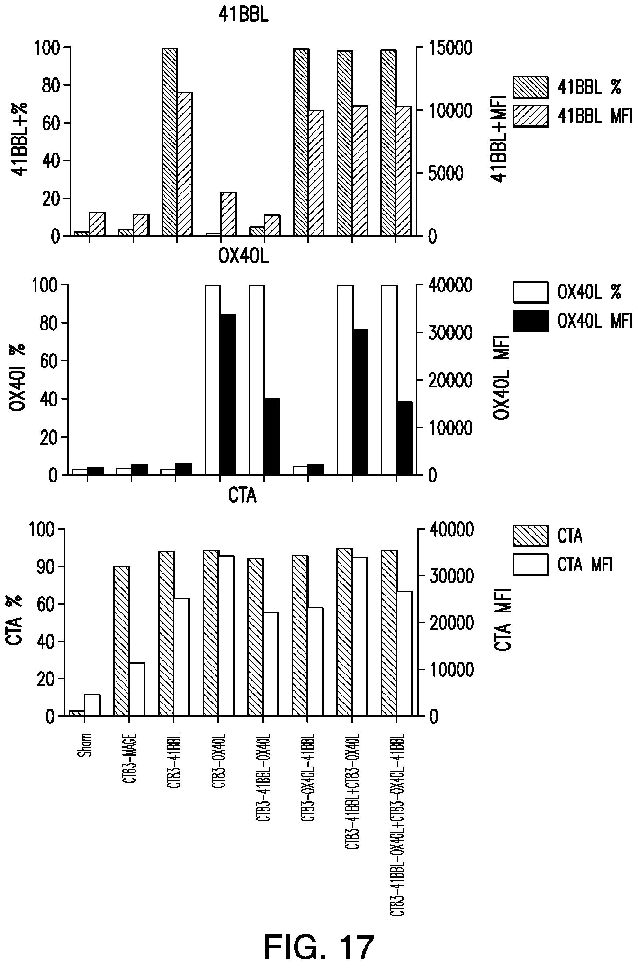

Figure 17 depicts co-expression of target tumor antigen with costimulatory molecules by vectors. Monocyte derived dendritic cells were transfected with RNA encoding a tumor antigen as a model antigen of interest and the costimulatory ligand 41BBL or OX40L alone or in tandem. Bar graphs indicate frequency and mean fluorescence intensity (MFI) of the level of expression of 4-1BBL (top), OX40L (middle) and tumor antigen (bottom) at 24 hours post-transfection. Co-electroporation of 4-1BBL and OX40L containing mini-genes did not compromise antigen expression.

### DETAILED DESCRIPTION OF THE INVENTION

### I. Definitions

Unless defined otherwise, all technical and scientific terms used herein have the meaning commonly understood by a person skilled in the art to which this invention belongs. The following references provide one of skill with a general definition of many of the terms used in this invention: Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al. (eds.), Springer Verlag (1991); and Hale & Marham, The Harper Collins Dictionary of Biology (1991). As used herein, the following terms have the meanings ascribed to them below, unless specified otherwise.

As used herein, the term "about" or "approximately" means within an acceptable error range for the particular value as determined by one of ordinary skill in the art, which will depend in part on how the value is measured or determined, *i.e*., the limitations of the measurement system. For example, "about" can mean within 3 or more than 3 standard deviations, per the practice in the art. Alternatively, "about" can mean a range of up to 20%, preferably up to 10%, more preferably up to 5%, and more preferably still up to 1% of a given value. Alternatively, particularly with respect to biological systems or processes, the term can mean within an order of magnitude, preferably within 5-fold, and more preferably within 2-fold, of a value.

As used herein, the term "cell population" refers to a group of at least two cells expressing similar or different phenotypes. In non-limiting examples, a cell population can include at least about 10, at least about 100, at least about 200, at least about 300, at least about 400, at least about 500, at least about 600, at least about 700, at least about 800, at least about 900, at least about 1000 cells expressing similar or different phenotypes.

As used herein, the term "vector" refers to any genetic element, such as a plasmid, phage, transposon, cosmid, chromosome, virus, virion, etc., which is capable of replication when associated with the proper control elements and which can transfer gene sequences into cells. Thus, the term includes cloning and expression vehicles, as well as viral vectors and plasmid vectors.

As used herein, the term "expression vector" refers to a recombinant nucleic acid sequence, *e*.*g*., a recombinant DNA molecule, containing a desired coding sequence and appropriate nucleic acid sequences necessary for the expression of the operably linked coding sequence in a particular host organism. Nucleic acid sequences necessary for expression in prokaryotes usually include a promoter, an operator (optional), and a ribosome binding site, often along with other sequences. Eukaryotic cells are known to utilize promoters, enhancers, and termination and polyadenylation signals.

Nucleic acid molecules useful in the presently disclosed subject matter include any nucleic acid molecule that encodes a polypeptide or a fragment thereof. In certain embodiments, nucleic acid molecules useful in the presently disclosed subject matter include nucleic acid molecules that encode a TCR or a target-binding portion thereof. Such nucleic acid molecules need not be 100% identical with an endogenous nucleic acid sequence, but will typically exhibit substantial identity. Polynucleotides having "substantial homology" or "substantial identity" to an endogenous sequence are typically capable of hybridizing with at least one strand of a double-stranded nucleic acid molecule.

By "hybridize" is meant pair to form a double-stranded molecule between complementary polynucleotide sequences (*e*.*g*., a gene described herein), or portions thereof, under various conditions of stringency. (*See, e.g.*, Wahl, G. M. and S. L. Berger (1987) Methods Enzymol. 152:399; Kimmel, A. R. (1987) Methods Enzymol. 152:507).

For example, stringent salt concentration will ordinarily be less than about 750 mM NaCl and 75 mM trisodium citrate, preferably less than about 500 mM NaCl and 50 mM trisodium citrate, and more preferably less than about 250 mM NaCl and about 25 mM trisodium citrate. Low stringency hybridization can be obtained in the absence of organic solvent, *e*.*g*., formamide, while high stringency hybridization can be obtained in the presence of at least about 35% formamide, and more preferably at least about 50% formamide. Stringent temperature conditions will ordinarily include temperatures of at least about 30°C, more preferably of at least about 37°C, and most preferably of at least about 42°C. Varying additional parameters, such as hybridization time, the concentration of detergent, *e*.*g*., sodium dodecyl sulfate (SDS), and the inclusion or exclusion of carrier DNA, are well known to those skilled in the art. Various levels of stringency are accomplished by combining these various conditions as needed. In certain embodiments, hybridization will occur at 30°C in 750 mM NaCl, 75 mM trisodium citrate, and 1% SDS. In certain embodiments, hybridization will occur at 37°C in 500 mM NaCl, 50 mM trisodium citrate, 1% SDS, 35% formamide, and 100 µg/ml denatured salmon sperm DNA (ssDNA). In certain embodiments, hybridization will occur at 42°C in 250 mM NaCl, 25 mM trisodium citrate, 1% SDS, 50% formamide, and 200 µg/ml ssDNA. Useful variations on these conditions will be readily apparent to those skilled in the art.

For most applications, washing steps that follow hybridization will also vary in stringency. Wash stringency conditions can be defined by salt concentration and by temperature. As above, wash stringency can be increased by decreasing salt concentration or by increasing temperature. For example, stringent salt concentration for the wash steps will preferably be less than about 30 mM NaCl and 3 mM trisodium citrate, and most preferably less than about 15 mM NaCl and 1.5 mM trisodium citrate. Stringent temperature conditions for the wash steps will ordinarily include a temperature of at least about 25°C, more preferably of at least about 42°C, and even more preferably of at least about 68°C. In certain embodiments, wash steps will occur at 25° C in 30 mM NaCl, 3 mM trisodium citrate, and 0.1% SDS. In certain embodiments, wash steps will occur at 42° C. in 15 mM NaCl, 1.5 mM trisodium citrate, and 0.1% SDS. In certain embodiments, wash steps will occur at 68°C in 15 mM NaCl, 1.5 mM trisodium citrate, and 0.1% SDS. Additional variations on these conditions will be readily apparent to those skilled in the art. Hybridization techniques are well known to those skilled in the art and are described, for example, in Benton and Davis (Science 196:180, 1977); Grunstein and Rogness (Proc. Natl. Acad. Sci., USA 72:3961, 1975); Ausubel et al. (Current Protocols in Molecular Biology, Wiley Interscience, New York, 2001); Berger and Kimmel (Guide to Molecular Cloning Techniques, 1987, Academic Press, New York); and Sambrook et al., Molecular Cloning: A Laboratory Manual, Cold Spring Harbor Laboratory Press, New York.

The terms "substantially homologous" or "substantially identical" mean a polypeptide or nucleic acid molecule that exhibits at least 50% homology or identity to a reference amino acid sequence (for example, any one of the amino acid sequences described herein) or nucleic acid sequence (for example, any one of the nucleic acid sequences described herein). For example, such a sequence is at least about 60%, about 65%, about 70%, about 75%, about 80%, about 85%, about 90%, about 95% or even about 99% homologous or identical at the amino acid level or nucleic acid to the sequence used for comparison.

Sequence homology or sequence identity is typically measured using sequence analysis software (for example, Sequence Analysis Software Package of the Genetics Computer Group, University of Wisconsin Biotechnology Center, 1710 University Avenue, Madison, Wis. 53705, BLAST, BESTFIT, GAP, or PILEUP/PRETTYBOX programs). Such software matches identical or similar sequences by assigning degrees of homology to various substitutions, deletions, and/or other modifications. In an exemplary approach to determining the degree of identity, a BLAST program may be used, with a probability score between e⁻³ and ^{e-100} indicating a closely related sequence.

As used herein, the term "analog" refers to a structurally related polypeptide or nucleic acid molecule having the function of a reference polypeptide or nucleic acid molecule.

As used herein, the term "ligand" refers to a molecule that binds to a receptor. In particular, the ligand binds a receptor on another cell, allowing for cell-to-cell recognition and/or interaction.

As used herein, the term "disease" refers to any condition or disorder that damages or interferes with the normal function of a cell, tissue, or organ. Examples of diseases include neoplasm or pathogen infection of cell.

An "effective amount" (or "therapeutically effective amount") is an amount sufficient to affect a beneficial or desired clinical result upon treatment. An effective amount can be administered to a subject in one or more doses. In terms of treatment, an effective amount is an amount that is sufficient to palliate, ameliorate, stabilize, reverse or slow the progression of the disease (*e*.*g*., a neoplasm), or otherwise reduce the pathological consequences of the disease (*e*.*g*., a neoplasm). The effective amount is generally determined by the physician on a case-by-case basis and is within the skill of one in the art. Several factors are typically taken into account when determining an appropriate dosage to achieve an effective amount. These factors include age, sex and weight of the subject, the condition being treated, the severity of the condition and the form and effective concentration of the immunoresponsive cells administered.

As used herein, the term "neoplasm" refers to a disease characterized by the pathological proliferation of a cell or tissue and its subsequent migration to or invasion of other tissues or organs. Neoplasm growth is typically uncontrolled and progressive, and occurs under conditions that would not elicit, or would cause cessation of, multiplication of normal cells. Neoplasms can affect a variety of cell types, tissues, or organs, including but not limited to an organ selected from the group consisting of bladder, colon, bone, brain, breast, cartilage, glia, esophagus, fallopian tube, gallbladder, heart, intestines, kidney, liver, lung, lymph node, nervous tissue, ovaries, pleura, pancreas, prostate, skeletal muscle, skin, spinal cord, spleen, stomach, testes, thymus, thyroid, trachea, urogenital tract, ureter, urethra, uterus, and vagina, or a tissue or cell type thereof. Neoplasms include cancers, such as sarcomas, carcinomas, or plasmacytomas (malignant tumor of the plasma cells).

As used herein, the term "heterologous nucleic acid molecule or polypeptide" refers to a nucleic acid molecule (*e*.*g*., a cDNA, DNA or RNA molecule) or polypeptide that is not normally present in a cell or sample obtained from a cell. This nucleic acid may be from another organism, or it may be, for example, an mRNA molecule that is not normally expressed in a cell or sample.

As used herein, the term "immunoresponsive cell" refers to a cell that functions in an immune response or a progenitor, or progeny thereof.

As used herein, the term "modulate" refers positively or negatively alter. Exemplary modulations include an about 1%, about 2%, about 5%, about 10%, about 25%, about 50%, about 75%, or about 100% change.

As used herein, the term "increase" refers to alter positively by at least about 5%, including, but not limited to, alter positively by about 5%, by about 10%, by about 25%, by about 30%, by about 50%, by about 75%, or by about 100%.

As used herein, the term "reduce" refers to alter negatively by at least about 5% including, but not limited to, alter negatively by about 5%, by about 10%, by about 25%, by about 30%, by about 50%, by about 75%, or by about 100%.

As used herein, the term "isolated cell" refers to a cell that is separated from the molecular and/or cellular components that naturally accompany the cell.

As used herein, the term "isolated," "purified," or "biologically pure" refers to material that is free to varying degrees from components which normally accompany it as found in its native state. "Isolate" denotes a degree of separation from original source or surroundings. "Purify" denotes a degree of separation that is higher than isolation. A "purified" or "biologically pure" protein is sufficiently free of other materials such that any impurities do not materially affect the biological properties of the protein or cause other adverse consequences. That is, a nucleic acid or polypeptide of the presently disclosed subject matter is purified if it is substantially free of cellular material, viral material, or culture medium when produced by recombinant DNA techniques, or chemical precursors or other chemicals when chemically synthesized. Purity and homogeneity are typically determined using analytical chemistry techniques, for example, polyacrylamide gel electrophoresis or high performance liquid chromatography. The term "purified" can denote that a nucleic acid or protein gives rise to essentially one band in an electrophoretic gel. For a protein that can be subjected to modifications, for example, phosphorylation or glycosylation, different modifications may give rise to different isolated proteins, which can be separately purified.

As used herein, the term "secreted" is meant a polypeptide that is released from a cell via the secretory pathway through the endoplasmic reticulum, Golgi apparatus, and as a vesicle that transiently fuses at the cell plasma membrane, releasing the proteins outside of the cell.

As used herein, the term "specifically binds" or "specifically binds to" or "specifically target" is meant a polypeptide or fragment thereof that recognizes and binds a biological molecule of interest (*e*.*g*., a polypeptide), but which does not substantially recognize and bind other molecules in a sample.

As used herein, the term "treating" or "treatment" refers to clinical intervention in an attempt to alter the disease course of the individual or cell being treated, and can be performed either for prophylaxis or during the course of clinical pathology. Therapeutic effects of treatment include, without limitation, preventing occurrence or recurrence of disease, alleviation of symptoms, diminishment of any direct or indirect pathological consequences of the disease, preventing metastases, decreasing the rate of disease progression, amelioration or palliation of the disease state, and remission or improved prognosis. By preventing progression of a disease or disorder, a treatment can prevent deterioration due to a disorder in an affected or diagnosed subject or a subject suspected of having the disorder, but also a treatment may prevent the onset of the disorder or a symptom of the disorder in a subject at risk for the disorder or suspected of having the disorder.

As used herein, the term "subject" refers to any animal (*e*.*g*., a mammal), including, but not limited to, humans, non-human primates, rodents, and the like (*e*.*g*., which is to be the recipient of a particular treatment, or from whom cells are harvested).

### II. Methods for Identifying Antigen-Specific TCRs

The present invention provides an *in vitro* method for identifying a T cell receptor (TCR) that targets an antigen, comprising:
a) contacting a plurality of lymphocytes with a plurality of antigen presenting cells (APCs), wherein the APC comprises an antigen and at least one exogenous costimulatory ligand;
b) identifying a lymphocyte that is stimulated by the APC (APC-stimulated lymphocyte); and
c) identifying a TCR comprised by the APC-stimulated lymphocyte identified in b); wherein the lymphocytes and the APCs are from a population of peripheral blood mononuclear cells of a single donor, and optionally wherein
   (i) the APC is a mature APC,
   (ii) the APC is a monocyte-derived dendritic cell; and/or
   (iii) the lymphocyte is a T cell.

The lymphocytes can be obtained from a first donor (e.g., a population of peripheral blood mononuclear cells (PBMCs) of the donor). The APC cells can be obtained from a second donor (e.g., a population of PBMCs of the donor). In certain embodiments, the first donor and the second donor are the same. In certain embodiments, the peripheral blood mononuclear cells are molecularly typed. In certain embodiments, the HLA-DR and -DQ alleles of the PBMCs are determined by molecular typing. In certain embodiments, molecular typing comprises sequence based typing, which is used for high-resolution identification of alleles of HLA-A, -B, -C, - DRB 1, -DQB1 and -DPB1. In certain embodiments, sequence based typing uses PCR to amplify the locus of interest, and sanger sequencing is then used to determine the nucleotide sequence of the PCR product

In certain embodiments, the APC is a mature APC. In certain embodiments, the APC is a mature dendritic cell. In certain embodiments, the APC is a monocyte-derived dendritic cell. In certain embodiments, the maturation of the APC comprises contacting the APC with LPS and/or IFNγ. In certain embodiments, the monocyte-derived dendritic cells are exposed to LPS and IFNγ, e.g., for about 20 to about 24 hours in vitro to derive mature dendritic cells.

In certain embodiments, the antigen is a tumor antigen. Non-limiting examples of tumor antigens include carbonic anhydrase IX (CAlX), carcinoembryonic antigen (CEA), CD8, CD7, CD10, CD19, CD20, CD22, CD30, CD33, CLL1, CD34, CD38, CD41, CD44, CD49f, CD56, CD74, CD133, CD138, CD123, CD44V6, an antigen of a cytomegalovirus (CMV) infected cell (e.g., a cell surface antigen), epithelial glycoprotein-2 (EGP-2), epithelial glycoprotein-40 (EGP-40), epithelial cell adhesion molecule (EpCAM), receptor tyrosine-protein kinases erb-B2,3,4 (erb-B2,3,4), folate-binding protein (FBP), fetal acetylcholine receptor (AChR), folate receptor-α, Ganglioside G2 (GD2), Ganglioside G3 (GD3), human Epidermal Growth Factor Receptor 2 (HER-2), human telomerase reverse transcriptase (hTERT), Interleukin-13 receptor subunit alpha-2 (IL-13Rα2), κ-light chain, kinase insert domain receptor (KDR), Lewis Y (LeY), L1 cell adhesion molecule (L1CAM), melanoma antigen family A, 1 (MAGE-A1), Mucin 16 (MUC-16), Mucin 1 (MUC-1), Mesothelin (MSLN), ERBB2, MAGEA3, p53, MART-1,GP100, Proteinase3 (PR1), Tyrosinase, Survivin, hTERT, EphA2, NKG2D ligands, cancer-testis antigen NY-ESO-1, oncofetal antigen (h5T4), prostate stem cell antigen (PSCA), prostate-specific membrane antigen (PSMA), ROR1, tumor-associated glycoprotein 72 (TAG-72), vascular endothelial growth factor R2 (VEGF-R2), and Wilms tumor protein (WT-1), BCMA, NKCS1, EGF1R, EGFR-VIII, CD99, CD70, ADGRE2, CCR1, LILRB2, PRAME CCR4, CD5, CD3, TRBC1, TRBC2, TIM-3, Integrin B7, ICAM-1, CD70, Tim3, CLEC12A, ERBB, PIK3KC, BRAF and KRAS.

In certain embodiments, antigen is selected from a group of targets, including but not limited to, tumor associated antigens, cancer germline antigens, and mutated antigens or neoantigens. In certain embodiments, the tumor associated antigens are selected from the group consisting of MART-1, tyrosinase, CEA, and MUC-1. In certain embodiments, the cancer germline antigens are selected from the group consisting of MAGE, and NY-ESO. In certain embodiments, the mutated antigens or neoantigens are selected from the group consisting of PIK3KC, BRAF, and KRAS. In certain embodiments, the antigen is selected from the group consisting ofPIK3CA, BRAF, KRAS, MART-1, tyrosinase, CEA, MUC-1, MAGE, and NY-ESO. In certain embodiments, the antigen comprises a mutation. In certain embodiments, the mutation is a driver mutation. In certain embodiments, the mutation is a shared antigen. In certain embodiments, the mutation is a private neoepitope. In certain embodiments, the antigen is a small peptide derived from the tumor antigen. In certain embodiments, the peptide comprises fewer than 24 amino acid residues, e.g., 23, 22, 21, 20, 19, 18, 17, 16, 15, 14, 13, 12, 11, 10, 9, 8, or 7.

In certain embodiments, the antigen is a pathogen antigen, e.g., for use in treating and/or preventing a pathogen infection or other infectious disease, for example, in an immunocompromised subject. Non-limiting examples of pathogen includes a virus, bacteria, fungi, parasite and protozoa capable of causing disease.

Non-limiting examples of viruses include, *Retroviridae* (e.g. human immunodeficiency viruses, such as HIV-1 (also referred to as HDTV-III, LAVE or HTLV-III/LAV, or HIV-III; and other isolates, such as HIV-LP; *Picornaviridae* (e.g. polio viruses, hepatitis A virus; enteroviruses, human Coxsackie viruses, rhinoviruses, echoviruses); *Calciviridae* (e.g. strains that cause gastroenteritis); *Togaviridae* (e.g. equine encephalitis viruses, rubella viruses); *Flaviridae* (e.g. dengue viruses, encephalitis viruses, yellow fever viruses); *Coronoviridae* (e.g. coronaviruses); Rhabdoviridae (e.g. vesicular stomatitis viruses, rabies viruses); *Filoviridae* (e.g. ebola viruses); *Paramyxoviridae* (e.g. parainfluenza viruses, mumps virus, measles virus, respiratory syncytial virus); *Orthomyxoviridae* (e.g. influenza viruses); *Bungaviridae* (e.g. Hantaan viruses, bunga viruses, phleboviruses and Naira viruses); Arena viridae (hemorrhagic fever viruses); *Reoviridae* (e.g. reoviruses, orbiviurses and rotaviruses); *Birnaviridae*; *Hepadnaviridae* (Hepatitis B virus); *Parvovirida* (parvoviruses); *Papovaviridae* (papilloma viruses, polyoma viruses); *Adenoviridae* (most adenoviruses); *Herpesviridae* (herpes simplex virus (HSV) 1 and 2, varicella zoster virus, cytomegalovirus (CMV), herpes virus; *Poxviridae* (variola viruses, vaccinia viruses, pox viruses); and *Iridoviridae* (e.g. African swine fever virus); and unclassified viruses (e.g. the agent of delta hepatitis (thought to be a defective satellite of hepatitis B virus), the agents of non-A, non-B hepatitis (class 1 =internally transmitted; class 2 =parenterally transmitted (i.e. Hepatitis C); Norwalk and related viruses, and astroviruses).

Non-limiting examples of bacteria include *Pasteurella*, *Staphylococci*, *Streptococcus*, *Escherichia coli*, *Pseudomonas* species, and *Salmonella* species. Specific examples of infectious bacteria include but are not limited to, *Helicobacter pyloris*, *Borelia burgdorferi*, *Legionella pneumophilia, Mycobacteria sps* (e.g. *M. tuberculosis*, *M. avium*, *M. intracellulare, M. kansaii, M. gordonae), Staphylococcus aureus, Neisseria gonorrhoeae, Neisseria meningitidis, Listeria monocytogenes, Streptococcus pyogenes* (Group A Streptococcus), *Streptococcus agalactiae* (Group B Streptococcus), *Streptococcus* (viridans group), *Streptococcus faecalis*, *Streptococcus bovis*, *Streptococcus* (anaerobic sps.), *Streptococcus pneumoniae*, pathogenic *Campylobacter sp., Enterococcus sp., Haemophilus influenzae*, *Bacillus antracis, corynebacterium diphtheriae, corynebacterium sp., Erysipelothrix rhusiopathiae, Clostridium perfringers, Clostridium tetani, Enterobacter aerogenes, Klebsiella pneumoniae, Pasturella multocida, Bacteroides sp., Fusobacterium nucleatum, Streptobacillus moniliformis*, *Treponema pallidium*, *Treponema pertenue*, *Leptospira*, *Rickettsia*, and *Actinomyces israelli.*

In certain embodiments, the pathogen antigen is a viral antigen present in Cytomegalovirus (CMV), a viral antigen present in Epstein Barr Virus (EBV), a viral antigen present in Human Immunodeficiency Virus (HIV), or a viral antigen present in influenza virus.

In certain embodiments, the APC further comprises a costimulatory ligand. In certain embodiments, the costimulatory ligand is selected from the tumor necrosis factor (TNF) superfamily ligands or the immunoglobulin (Ig) superfamily ligands. In certain embodiments, the costimulatory ligand is selected from the group consisting of 41BBL, OX40L, CD30L, CD80, CD86, CD40L, ICOSL, CD70, and any combination thereof. In certain embodiments, the APC further comprises two costimulatory ligands.

In certain embodiments, the antigen and/or the costimulatory ligand are exogenous, e.g., transfected into the APC. In certain embodiments, the antigen and/or the costimulatory ligand are expressed through a vector. In certain embodiments, the vector is a recombinant RNA, a plasmid, or a viral vector. In certain embodiments, the antigen and/or the costimulatory ligand are transiently expressed in the APC. In certain embodiments, the antigen and/or the costimulatory ligand are stably expressed in the APC.

In certain embodiments, the costimulatory ligand and the antigen are constructed in a single, multicistronic expression cassette, in multiple expression cassettes of a single vector, or in multiple vectors. In certain embodiments, the costimulatory ligand and the antigen are connected by a polycistronic expression element. Examples of elements that create polycistronic expression cassette include, but is not limited to, various viral and non-viral Internal Ribosome Entry Sites (IRES, *e.g.*, FGF-1 IRES, FGF-2 IRES, VEGF IRES, IGF-II IRES, NF-κB IRES, RUNX1 IRES, p53 IRES, hepatitis A IRES, hepatitis C IRES, pestivirus IRES, aphthovirus IRES, picornavirus IRES, poliovirus IRES and encephalomyocarditis virus IRES) and cleavable linkers (*e.g.*, a furin peptide and a 2A peptide, *e*.*g*., furin-2A peptide, P2A, T2A, E2A and F2A peptides). In certain embodiments, the costimulatory ligand and the antigen are connected by a 2A peptide. In certain embodiments, the 2A peptide comprises the amino acid sequence set forth in SEQ ID NO: 1, which is provided below.

### RAKRSGSGATNFSLLKQAGDVEENPGP [SEQ ID NO: 1]

In certain embodiments, the 2A peptide comprise the amino acid sequence set forth in SEQ ID NO: 1. In certain embodiments, the nucleic acid sequence encoding the 2A peptide is codon-optimized. An exemplary codon-optimized nucleic acid sequence encoding the amino acid sequence of SEQ ID NO: 1 is set forth in SEQ ID NO: 2, which is provided below.

AGGGCAAAGAGGAGCGGATCCGGAGCCACCAATTTCTCTCTGCTGAA
GCAGGCAGGCGATGTGGAGGAGAACCCAGGACCT [SEQ ID NO: 2]

An exemplary non-codon-optimized nucleic acid sequence encoding the amino acid sequence of SEQ ID NO: 1 is set forth in SEQ ID NO: 3

AGGGCAAAGCGGAGCGGAAGTGGCGCCACCAATTTCTCCCTGCTGAA
ACAGGCCGGGGATGTCGAGGAAAATCCCGGACCT [SEQ ID NO: 3]

In certain embodiments, identifying an APC-stimulated lymphocyte comprises detecting an increase of a cytokine expression. In certain embodiments, identifying an APC-stimulated lymphocyte comprises detecting an increase of an expression of an acute costimulatory molecule, e.g., 4-BB andOX40, an increase of a cytokine secretion, an increase of a cytolytic capability against an antigen-expressing target, or a combination of the foregoing. In certain embodiments, the cytokine is selected from the group consisting of IL-2, IFNγ, TNFα, MIP1α, granzyme B, perforin, CD107a, CD107b, TGF-β, IL-4, IL-3, IL-6, IL-7, IL-11, IL-12, IL-15, IL-17, IL-18, IL-21 and any combination thereof. Any suitable methods for measuring protein expression can be used to measure the cytokine expression. In certain embodiments, the cytokine expression is measured by polymerase chain reaction (PCR), *e*.*g*., quantitative polymerase chain reaction (qPCR). In certain embodiments, the cytokine expression is measured by an enzyme linked immunosorbent assay (ELISA), *e.g.,* a sandwich ELISA. In certain embodiments, the cytokine is secreted. In certain embodiments, the APC-stimulated lymphocyte is capable of proliferating. In certain embodiments, the method comprises comparing the cytokine expression of the APC-stimulated lymphocyte with the cytokine expression of a lymphocyte that is contacted with an APC that does not comprise the antigen. In certain embodiments, the method comprises comparing the cytokine expressions of an APC-stimulated lymphocyte before and after contacting with an APC that comprises the antigen.

In certain embodiments, the mRNA, cDNA or the genomic DNA encoding the TCR is identified by nucleic acid sequencing. In certain embodiments, the nucleic acid sequencing comprises single cell sequencing.

In certain embodiments, the method further comprises validating an antigen-binding specificity of the TCR. In certain embodiments, validating the antigen-binding specificity of the TCR comprises:
i) expressing the TCR in a lymphocyte,
ii) contacting the lymphocyte in step i) with an APC comprising the antigen, and
iii) determining that the TCR is specific to the antigen when the lymphocyte in step ii) is stimulated compared to a control lymphocyte, wherein the control lymphocyte expresses the TCR and is contacted with an APC that does not comprise the antigen. In certain embodiments, the TCR is reconstructed into a construct. In certain embodiments, the stimulation of the lymphocyte is identified by detecting an increase of a cytokine expression. In certain embodiments, the cytokine is selected from the group consisting of IL-2, IFNγ, TNFα, MIP1α, granzyme B, perforin, CD107a, CD107b, TGF-β, IL-4, IL-3, IL-6, IL-7, IL-11, IL-12, IL-15, IL-17, IL-18, IL-21, and combinations thereof. In certain embodiments, the antigen is the full length sequence.

In certain embodiments, the lymphocyte is a T cell.

### III. T-cell Receptor (TCR)

A TCR is a disulfide-linked heterodimeric protein consisting of two variable chains expressed as part of a complex with the invariant CD3 chain molecules. A TCR is found on the surface of T cells, and is responsible for recognizing antigens as peptides bound to major histocompatibility complex (MHC) molecules. In certain embodiments, the method identifies a TCR disclosed herein comprising an α chain and a β chain (encoded by TRA and TRB, respectively). In certain embodiments, a TCR disclosed herein comprises a γ chain and a δ chain (encoded by TRG and TRD, respectively).

Each chain of a TCR comprises two extracellular domains: a variable region and a constant region. The constant region is proximal to the cell membrane, followed by a transmembrane domain and a short cytoplasmic tail (i.e., an intracellular domain). The variable region binds to the peptide/MHC complex. The variable region of both chains each has three complementarity determining regions (CDRs).

In certain embodiments, the method identifies a TCR that can form a receptor complex with three dimeric signaling modules CD3δ/ε, CD3γ/ε and CD247 ζ/ζ or ζ/η. When a TCR complex engages with its antigen and MHC (peptide/MHC), the T cell expressing the TCR complex is activated.

The TCR identified by the method of the present invention may be a recombinant TCR. The TCR may be a non-naturally occurring TCR. The TCR may differ from any naturally occurring TCR by at least one amino acid residue, such as by at least 2, 3, 4, 5, 6, 7, 8, 9, 10, 11, 12, 13, 14, 15, 20, 25, 30, 40, 50, 60, 70, 80, 90, 100 or more amino acid residues. The TCR may be modified from a naturally occurring TCR by at least one amino acid residue. The TCR may be modified from a naturally occurring TCR by at least 2, 3, 4, 5, 6, 7, 8, 9, 10, 11, 12, 13, 14, 15, 20, 25, 30, 40, 50, 60, 70, 80, 90, 100 or more amino acid residues.

The TCR may specifically target an antigen or an epitope thereof associated with a HLA class I complex, e.g., HLA-A, HLA-B and HLA-C. In certain embodiments, the TCR specifically targets an antigen or an epitope thereof associated with a HLA class II complex, e.g., HLA-DP, HLA-DM, HLA-DO, HLA-DQ and HLA-DR.

The TCR may for example target a tumor antigen or a pathogen antigen. Non-limiting examples of tumor antigens include carbonic anhydrase IX (CAlX), carcinoembryonic antigen (CEA), CD8, CD7, CD10, CD19, CD20, CD22, CD30, CD33, CLL1, CD34, CD38, CD41, CD44, CD49f, CD56, CD74, CD133, CD138, CD123, CD44V6, an antigen of a cytomegalovirus (CMV) infected cell (e.g., a cell surface antigen), epithelial glycoprotein-2 (EGP-2), epithelial glycoprotein-40 (EGP-40), epithelial cell adhesion molecule (EpCAM), receptor tyrosine-protein kinases erb-B2,3,4 (erb-B2,3,4), folate-binding protein (FBP), fetal acetylcholine receptor (AChR), folate receptor-α, Ganglioside G2 (GD2), Ganglioside G3 (GD3), human Epidermal Growth Factor Receptor 2 (HER-2), human telomerase reverse transcriptase (hTERT), Interleukin-13 receptor subunit alpha-2 (IL-13Rα2), κ-light chain, kinase insert domain receptor (KDR), Lewis Y (LeY), L1 cell adhesion molecule (L1CAM), melanoma antigen family A, 1 (MAGE-A1), Mucin 16 (MUC-16), Mucin 1 (MUC-1), Mesothelin (MSLN), ERBB2, MAGEA3, p53, MART-1,GP100, Proteinase3 (PR1), Tyrosinase, Survivin, hTERT, EphA2, NKG2D ligands, cancer-testis antigen NY-ESO-1, oncofetal antigen (h5T4), prostate stem cell antigen (PSCA), prostate-specific membrane antigen (PSMA), ROR1, tumor-associated glycoprotein 72 (TAG-72), vascular endothelial growth factor R2 (VEGF-R2), and Wilms tumor protein (WT-1), BCMA, NKCS1, EGF1R, EGFR-VIII, CD99, CD70, ADGRE2, CCR1, LILRB2, PRAME CCR4, CD5, CD3, TRBC1, TRBC2, TIM-3, Integrin B7, ICAM-1, CD70, Tim3, CLEC12A, ERBB, PIK3KC, BRAF and KRAS.

Targets may for example be tumor associated antigens, cancer germline antigens, and mutated antigens or neoantigens. Examples of tumor associated antigens are MART-1, tyrosinase, CEA, and MUC-1. Examples of cancer germline antigens are MAGE, and NY-ESO. Examples of mutated antigens or neoantigens are PIK3KC, BRAF, and KRAS. In certain embodiments, the antigen is selected from the group consisting of PIK3CA, BRAF, KRAS, MART-1, tyrosinase, CEA, MUC-1, MAGE, and NY-ESO. Where the antigen comprises a mutation, the mutation may be for example be a driver mutation, a shared antigen or a private neoepitope. The antigen may also be a small peptide derived from the tumor antigen such as a peptide comprising fewer than 24 amino acid residues, e.g., 23, 22, 21, 20, 19, 18, 17, 16, 15, 14, 13, 12, 11, 10, 9, 8, or 7.

The antigen identified by the method of the invention may be a pathogen antigen, and thus useful in treating and/or preventing a pathogen infection or other infectious disease, for example, in an immunocompromised subject. Non-limiting examples of pathogen includes a virus, bacteria, fungi, parasite and protozoa capable of causing disease.

Examples of viruses include, *Retroviridae* (e.g. human immunodeficiency viruses, such as HIV-1 (also referred to as HDTV-III, LAVE or HTLV-III/LAV, or HIV-III; and other isolates, such as HIV-LP; *Picornaviridae* (e.g. polio viruses, hepatitis A virus; enteroviruses, human Coxsackie viruses, rhinoviruses, echoviruses); *Calciviridae* (e.g. strains that cause gastroenteritis); *Togaviridae* (e.g. equine encephalitis viruses, rubella viruses); *Flaviridae* (e.g. dengue viruses, encephalitis viruses, yellow fever viruses); *Coronoviridae* (e.g. coronaviruses); Rhabdoviridae (e.g. vesicular stomatitis viruses, rabies viruses); *Filoviridae* (e.g. ebola viruses); *Paramyxoviridae* (e.g. parainfluenza viruses, mumps virus, measles virus, respiratory syncytial virus); *Orthomyxoviridae* (e.g. influenza viruses); *Bungaviridae* (e.g. Hantaan viruses, bunga viruses, phleboviruses and Naira viruses); Arena viridae (hemorrhagic fever viruses); *Reoviridae* (e.g. reoviruses, orbiviurses and rotaviruses); *Birnaviridae*; *Hepadnaviridae* (Hepatitis B virus); *Parvovirida* (parvoviruses); *Papovaviridae* (papilloma viruses, polyoma viruses); *Adenoviridae* (most adenoviruses); *Herpesviridae* (herpes simplex virus (HSV) 1 and 2, varicella zoster virus, cytomegalovirus (CMV), herpes virus; *Poxviridae* (variola viruses, vaccinia viruses, pox viruses); and *Iridoviridae* (e.g. African swine fever virus); and unclassified viruses (e.g. the agent of delta hepatitis (thought to be a defective satellite of hepatitis B virus), the agents of non-A, non-B hepatitis (class 1 =internally transmitted; class 2 =parenterally transmitted (i.e. Hepatitis C); Norwalk and related viruses, and astroviruses).

Examples of bacteria include *Pasteurella*, *Staphylococci*, *Streptococcus*, *Escherichia coli*, *Pseudomonas* species, and *Salmonella* species. Specific examples of infectious bacteria include but are not limited to, *Helicobacter pyloris*, *Borelia burgdorferi*, *Legionella pneumophilia*, *Mycobacteria sps* (e.g. *M. tuberculosis*, *M. avium*, *M. intracellulare, M. kansaii, M. gordonae), Staphylococcus aureus, Neisseria gonorrhoeae, Neisseria meningitidis*, *Listeria monocytogenes*, *Streptococcus pyogenes* (Group A Streptococcus), *Streptococcus agalactiae* (Group B Streptococcus), *Streptococcus* (viridans group), *Streptococcus faecalis, Streptococcus bovis, Streptococcus* (anaerobic sps.), *Streptococcus pneumoniae,* pathogenic *Campylobacter sp., Enterococcus sp., Haemophilus influenzae, Bacillus antracis, corynebacterium diphtheriae, corynebacterium sp., Erysipelothrix rhusiopathiae, Clostridium perfringers, Clostridium tetani, Enterobacter aerogenes, Klebsiella pneumoniae, Pasturella multocida, Bacteroides sp., Fusobacterium nucleatum, Streptobacillus moniliformis, Treponema pallidium, Treponema pertenue, Leptospira, Rickettsia,* and *Actinomyces israelli.*

The pathogen antigen may be a viral antigen present in Cytomegalovirus (CMV), a viral antigen present in Epstein Barr Virus (EBV), a viral antigen present in Human Immunodeficiency Virus (HIV), or a viral antigen present in influenza virus.

The TCR identified by the method of the invention may be constructed into a vector, such as a vector further comprises a promoter, *e.g.*, a constitutive promoter or an inducible promoter, for expressing nucleic acid sequences in human cells. Promoters for use in expressing TCR genes can be a constitutive promoter, such as ubiquitin C (UbiC) promoter.

Disclosed is further an isolated nucleic acid molecule encoding a TCR identified by the method of the invention or a functional portion thereof. The isolated nucleic acid molecule may encode both an α chain and a β chain of a TCR. The α chain and the β chain may be separated by a self-cleavage peptide, *e.g.*, a 2A-peptide. The α chain and the β chain may be separated by a furin-2A-peptide, *e*.*g*., a peptide set forth in SEQ ID NO: 1.

### IV. Immunoresponsive Cells

### Immunoresponsive Cells expressing TCR

The antigen-specific TCR identified by the method of the invention may be introduced in in cells. Such cells are useful for treating and/or preventing a malignancy in human subjects, *e*.*g*., breast cancer.

The immunoresponsive cells can be transduced with a TCR identified by the method of the invention such that the cells express the TCR, which may be useful for the treatment of a malignancy, *e*.*g*., breast cancer.

The immunoresponsive cells can be cells of the lymphoid lineage. The lymphoid lineage, comprising lymphocytes, e.g., B, T and natural killer (NK) cells, provides for the production of TCRs, regulation of the cellular immune system, detection of foreign agents in the blood, detection of cells foreign to the host, and the like. Non-limiting examples of immunoresponsive cells of the lymphoid lineage include T cells, Natural Killer (NK) cells, embryonic stem cells, and pluripotent stem cells (*e.g.*, those from which lymphoid cells may be differentiated). T cells can be lymphocytes that mature in the thymus and are chiefly responsible for cell-mediated immunity. T cells are involved in the adaptive immune system. The T cells of the presently disclosed subject matter can be any type of T cells, including, but not limited to, T helper cells, cytotoxic T cells, memory T cells (including central memory T cells, stem-cell-like memory T cells (or stem-like memory T cells), and two types of effector memory T cells: *e*.*g*., T_{EM} cells and T_{EMRA} cells, Regulatory T cells (also known as suppressor T cells), Natural killer T cells, Mucosal associated invariant T cells, and γδ T cells. Cytotoxic T cells (CTL or killer T cells) are a subset of T lymphocytes capable of inducing the death of infected somatic or tumor cellsThe TCR-expressing T cells may for example express Foxp3 to achieve and maintain a T regulatory phenotype.

Natural killer (NK) cells can be lymphocytes that are part of cell-mediated immunity and act during the innate immune response. NK cells do not require prior activation in order to perform their cytotoxic effect on target cells.

The immunoresponsive cells can express a TCR that specifically binds to an antigen of interest (*e.g.*, a tumor antigen), for the treatment of cancer, *e*.*g*., breast cancer. Such immunoresponsive cells can be for administration to a subject (*e*.*g*., a human subject) in need thereof for the treatment of cancer, *e*.*g*., breast cancer. The immunoresponsive cell may be a T cell. The T cell can be a CD4⁺ T cell or a CD8⁺ T cell.

The immunoresponsive cell can further include at least one recombinant or exogenous co-stimulatory ligand. For example, a immunoresponsive cell can be further transduced with at least one co-stimulatory ligand, such that the immunoresponsive cell co-expresses or is induced to co-express the TCR and the at least one co-stimulatory ligand. The interaction between the TCR and at least one co-stimulatory ligand provides a non-antigen-specific signal important for full activation of an immunoresponsive cell (*e*.*g*., T cell). Co-stimulatory ligands include, but are not limited to, members of the tumor necrosis factor (TNF) superfamily, and immunoglobulin (Ig) superfamily ligands. TNF is a cytokine involved in systemic inflammation and stimulates the acute phase reaction. Its primary role is in the regulation of immune cells. Members of TNF superfamily share a number of common features. The majority of TNF superfamily members are synthesized as type II transmembrane proteins (extracellular C-terminus) containing a short cytoplasmic segment and a relatively long extracellular region. TNF superfamily members include, without limitation, nerve growth factor (NGF), CD40L (CD40L)/CD154, CD137L/4-1BBL, TNF-α, CD134L/OX40L/CD252, CD27L/CD70, Fas ligand (FasL), CD30L/CD153, tumor necrosis factor beta (TNFβ)/lymphotoxin-alpha (LTα), lymphotoxin-beta (LTβ), CD257B cell-activating factor (BAFF)Blys/THANK/Tall-1, glucocorticoid-induced TNF Receptor ligand (GITRL), and TNF-related apoptosis-inducing ligand (TRAIL), LIGHT (TNFSF14). The immunoglobulin (Ig) superfamily is a large group of cell surface and soluble proteins that are involved in the recognition, binding, or adhesion processes of cells. These proteins share structural features with immunoglobulins -- they possess an immunoglobulin domain (fold). Immunoglobulin superfamily ligands include, but are not limited to, CD80 and CD86, both ligands for CD28, PD-L1/(B7-H1) that ligands for PD-1. The at least one co-stimulatory ligand may for eample be 4-1BBL, CD80, CD86, CD70, OX40L, CD48, TNFRSF14, PD-L1, or combinations thereof. The immunoresponsive cell may comprises one recombinant co-stimulatory ligand that is 4-1BBL or two recombinant co-stimulatory ligands that are 4-1BBL and CD80.

Furthermore, a immunoresponsive cell can further comprise at least one exogenous cytokine. For example, a presently disclosed immunoresponsive cell can be further transduced with at least one cytokine, such that the immunoresponsive cell secretes the at least one cytokine as well as expresses the TCR. The at least one cytokine may for example be IL-2, IL-3, IL-6, IL-7, IL-11, IL-12, IL-15, IL-17, or IL-21.

The human lymphocytes that can be used in peripheral donor lymphocytes are disclosed in Sadelain, M., et al. 2003 Nat Rev Cancer 3:35-45 (disclosing peripheral donor lymphocytes genetically modified to express TCRs), in Morgan, R.A., et al. 2006 Science 314:126-129 (disclosing peripheral donor lymphocytes genetically modified to express a full-length tumor antigen-recognizing T cell receptor complex comprising the α and β heterodimer), in Panelli, M.C., et al. 2000 J Immunol 164:495-504; Panelli, M.C., et al. 2000 J Immunol 164:4382-4392 (disclosing lymphocyte cultures derived from tumor infiltrating lymphocytes (TILs) in tumor biopsies), and in Dupont, J., et al. 2005 Cancer Res 65:5417-5427; Papanicolaou, G.A., et al. 2003 Blood 102:2498-2505 (disclosing selectively *in* vitro-expanded antigen-specific peripheral blood leukocytes employing artificial antigen-presenting cells (AAPCs) or pulsed dendritic cells). The immunoresponsive cells (*e.g.*, T cells) can be autologous, non-autologous (*e*.*g*., allogeneic), or derived *in vitro* from engineered progenitor or stem cells.

The unpurified source of CTLs may be any known in the art, such as the bone marrow, fetal, neonate or adult or other hematopoietic cell source, *e*.*g*., fetal liver, peripheral blood or umbilical cord blood. Various techniques can be employed to separate the cells. For instance, negative selection methods can remove non-CTLs initially.

A large proportion of terminally differentiated cells can be initially removed by a relatively crude separation. For example, magnetic bead separations can be used initially to remove large numbers of irrelevant cells. Preferably, at least about 80%, usually at least about 70% of the total hematopoietic cells will be removed prior to cell isolation.

Procedures for separation include density gradient centrifugation; resetting; coupling to particles that modify cell density; magnetic separation with TCR-coated magnetic beads; affinity chromatography; cytotoxic agents joined to or used in conjunction with a mAb, including, but not limited to, complement and cytotoxins; and panning with TCR attached to a solid matrix, *e*.*g*. plate, chip, elutriation or any other convenient technique.

Techniques for separation and analysis include flow cytometry, which can have varying degrees of sophistication, *e*.*g*., a plurality of color channels, low angle and obtuse light scattering detecting channels, impedance channels.

The cells can be selected against dead cells, by employing dyes associated with dead cells such as propidium iodide (PI). Preferably, the cells are collected in a medium comprising 2% fetal calf serum (FCS) or 0.2% bovine serum albumin (BSA) or any other suitable, preferably sterile, isotonic medium.

### Antigen Presenting Cells (APCs)

The antigen presenting cells (APCs) comprising an antigen of interest, *e.g.*, a tumor antigen disclosed herein can be used in the method of the present inventionto identify an antigen-specific TCR. An APC can be transduced with an antigen of interest such that the cells express the TCR.

Any cell capable of presenting an antigen of interest on the cell surface can be an APC. The antigen may be presented in a major histocompatibility complex (MHC). The APC may be a professional APC, *e.g.*, a dendritic cell, a macrophages or a B cell. The APC may be a non-professional APC. The APC may be a cell of the lymphoid lineage disclosed herein, *e.g.*, a B cell. The APC may be a cell of the myeloid lineage, *e.g.,* a dendritic cell or a macrophage. The APC may be derived from myeloblast, *e*.*g*., a monocyte. In certain embodiments, the APC is a monocyte-derived dendritic cell.

The APC may be a mature APC,such as a mature dendritic cell or a mature monocyte-derived dendritic cell. The maturation of the APC may comprise contacting the APC with LPS and/or IFNγ. For example, the monocyte-derived dendritic cells are exposed to LPS and IFNγ, e.g., for about 20 to about 24 hours in vitro to derive mature dendritic cells.

The APC further comprises a costimulatory ligand. The costimulatory ligand may for example be from the tumor necrosis factor (TNF) superfamily ligands or the immunoglobulin (Ig) superfamily ligands. For example, the costimulatory ligand may be 41BBL, OX40L, CD30L, CD80, CD86, CD40L, ICOSL, CD70, or combinations thereof. The APC further comprises two costimulatory ligands, e.g., 4-1BBL and OX40L.

The at least one costimulatory ligand is exogenous, e.g., transfected into the APC. In one example, the antigen and at least two costimulatory ligands are co-transfected into the APC. The antigen and/or the costimulatory may be expressed through a vector, such as for example a recombinant RNA, a plasmid, or a viral vector. The antigen and/or the costimulatory ligand may be transiently or stably expressed in the APC.

The costimulatory ligand and the antigen may for example constructed in a single, multicistronic expression cassette, in multiple expression cassettes of a single vector, or in multiple vectors. The costimulatory ligand and the antigen may for example be connected by a polycistronic expression element. Examples of elements that create polycistronic expression cassette include various viral and non-viral Internal Ribosome Entry Sites (IRES, *e.g.*, FGF-1 IRES, FGF-2 IRES, VEGF IRES, IGF-II IRES, NF-κB IRES, RUNX1 IRES, p53 IRES, hepatitis A IRES, hepatitis C IRES, pestivirus IRES, aphthovirus IRES, picornavirus IRES, poliovirus IRES and encephalomyocarditis virus IRES) and cleavable linkers (*e.g.*, a furin peptide and a 2A peptide, *e.g.*, furin-2A peptide, P2A, T2A, E2A and F2A peptides). The 2A peptide may for example comprise the amino acid sequence set forth in SEQ ID NO: 1. In another example, the 2A peptide comprise the amino acid sequence set forth in SEQ ID NO: 1. The nucleic acid sequence encoding the 2A peptide may be codon-optimized. An exemplary codon-optimized nucleic acid sequence encoding the amino acid sequence of SEQ ID NO: 1 is set forth in SEQ ID NO: 2.

### V. Vectors

Genetic modification of immunoresponsive cells (*e.g.*, T cells, NK cells) can be accomplished by transducing a substantially homogeneous cell composition with a recombinant DNA or RNA construct. The vector can be a retroviral vector (*e*.*g*., gamma retroviral), which is employed for the introduction of the DNA or RNA construct into the host cell genome. For example, a polynucleotide encoding a TCR disclosed herein can be cloned into a retroviral vector and expression can be driven from its endogenous promoter, from the retroviral long terminal repeat, or from an alternative internal promoter.

Non-viral vectors or RNA may be used as well. Random chromosomal integration, or targeted integration (*e*.*g*., using a nuclease, transcription activator-like effector nucleases (TALENs), Zinc-finger nucleases (ZFNs), and/or clustered regularly interspaced short palindromic repeats (CRISPRs), or transgene expression (*e*.*g*., using a natural or chemically modified RNA) can be used.

For initial genetic modification of the cells to provide a TCR expressing cells, a retroviral vector is generally employed for transduction, however any other suitable viral vector or non-viral delivery system can be used. For subsequent genetic modification of the cells to provide cells comprising an antigen presenting complex comprising at least two co-stimulatory ligands, retroviral gene transfer (transduction) likewise proves effective. Combinations of retroviral vector and an appropriate packaging line are also suitable, where the capsid proteins will be functional for infecting human cells. Various amphotropic virus-producing cell lines are known, including, but not limited to, PA12 (Miller, et al. (1985) Mol. Cell. Biol. 5:431-437); PA317 (Miller, et al. (1986) Mol. Cell. Biol. 6:2895-2902); and CRIP (Danos, et al. (1988) Proc. Natl. Acad. Sci. USA 85:6460-6464). Non -amphotropic particles are suitable too, *e*.*g*., particles pseudotyped with VSVG, RD114 or GALV envelope and any other known in the art.

Possible methods of transduction also include direct co-culture of the cells with producer cells, *e.g.*, by the method of Bregni, et al. (1992) Blood 80:1418-1422, or culturing with viral supernatant alone or concentrated vector stocks with or without appropriate growth factors and polycations, *e*.*g*., by the method of Xu, et al. (1994) Exp. Hemat. 22:223-230; and Hughes, et al. (1992) J. Clin. Invest. 89:1817.

Transducing viral vectors can be used to express a co-stimulatory ligand and/or secrets a cytokine (*e*.*g*., 4-1BBL and/or IL-12) in an immunoresponsive cell. Preferably, the chosen vector exhibits high efficiency of infection and stable integration and expression (*see, e.g.*, Cayouette et al., Human Gene Therapy 8:423-430, 1997; Kido et al., Current Eye Research 15:833-844, 1996; Bloomer et al., Journal of Virology 71:6641-6649, 1997; Naldini et al., Science 272:263 267, 1996; and Miyoshi et al., Proc. Natl. Acad. Sci. U.S.A. 94:10319, 1997). Other viral vectors that can be used include, for example, adenoviral, lentiviral, and adeno-associated viral vectors, vaccinia virus, a bovine papilloma virus, or a herpes virus, such as Epstein-Barr Virus (also see, for example, the vectors of Miller, Human Gene Therapy 15-14, 1990; Friedman, Science 244:1275-1281, 1989; Eglitis et al., BioTechniques 6:608-614, 1988; Tolstoshev et al., Current Opinion in Biotechnology 1:55-61, 1990; Sharp, The Lancet 337:1277-1278, 1991; Cornetta et al., Nucleic Acid Research and Molecular Biology 36:311-322, 1987; Anderson, Science 226:401-409, 1984; Moen, Blood Cells 17:407-416, 1991; Miller et al., Biotechnology 7:980-990, 1989; Le Gal La Salle et al., Science 259:988-990, 1993; and Johnson, Chest 107:77S- 83S, 1995). Retroviral vectors are particularly well developed and have been used in clinical settings (Rosenberg et al., N. Engl. J. Med 323:370, 1990; Anderson et al., U.S. Pat. No. 5,399,346). In one example, the vector expressing a presently disclosed TCR is a retroviral vector, *e*.*g*., an oncoretroviral vector.

Non-viral approaches can also be employed for the expression of a protein in cell. For example, a nucleic acid molecule can be introduced into a cell by administering the nucleic acid in the presence of lipofection (Feigner et al., Proc. Nat'l. Acad. Sci. U.S.A. 84:7413, 1987; Ono et al., Neuroscience Letters 17:259, 1990; Brigham et al., Am. J. Med. Sci. 298:278, 1989; Staubinger et al., Methods in Enzymology 101:512, 1983), asialoorosomucoid-polylysine conjugation (Wu et al., Journal of Biological Chemistry 263:14621 , 1988; Wu et al., Journal of Biological Chemistry 264:16985, 1989), or by micro-injection under surgical conditions (Wolff et al., Science 247:1465, 1990). Other non-viral means for gene transfer include transfection *in vitro* using calcium phosphate, DEAE dextran, electroporation, and protoplast fusion. Liposomes can also be potentially beneficial for delivery of DNA into a cell. Transplantation of normal genes into the affected tissues of a subject can also be accomplished by transferring a normal nucleic acid into a cultivatable cell type *ex vivo* (*e.g.*, an autologous or heterologous primary cell or progeny thereof), after which the cell (or its descendants) are injected into a targeted tissue or are injected systemically. Recombinant receptors can also be derived or obtained using transposases or targeted nucleases (*e*.*g*., Zinc finger nucleases, meganucleases, or TALE nucleases). Transient expression may be obtained by RNA electroporation.

cDNA expression for use in polynucleotide therapy methods can be directed from any suitable promoter (*e*.*g*., the human cytomegalovirus (CMV), simian virus 40 (SV40), or metallothionein promoters), and regulated by any appropriate mammalian regulatory element or intron (*e*.*g*., the elongation factor 1α enhancer/promoter/intron structure). For example, if desired, enhancers known to preferentially direct gene expression in specific cell types can be used to direct the expression of a nucleic acid. The enhancers used can include, without limitation, those that are characterized as tissue- or cell-specific enhancers. Alternatively, if a genomic clone is used as a therapeutic construct, regulation can be mediated by the cognate regulatory sequences or, if desired, by regulatory sequences derived from a heterologous source, including any of the promoters or regulatory elements described above.

The resulting cells can be grown under conditions similar to those for unmodified cells, whereby the modified cells can be expanded and used for a variety of purposes.

### VI. Suitable routes for administration

The TCRs and immunoresponsive cells comprising thereof can be for administration systemically or directly to a subject for treating or preventing a neoplasm. For example, the TCRs and immunoresponsive cells comprising thereof are for direct injection into an organ of interest (*e*.*g*., an organ affected by a neoplasm). Alternatively or additionally, the TCRs and immunoresponsive cells comprising thereof are for indirect administration to the organ of interest, for example, for administration into the circulatory system (*e*.*g*., the tumor vasculature). Expansion and differentiation agents can be provided prior to, during or after administration of cells and compositions to increase production of T cells *in vitro or in vivo.*

The TCRs and immunoresponsive cells comprising thereof of the presently disclosed subject matter can be for administration in any physiologically acceptable vehicle, normally intravascularly, although they may also be introduced into bone or other convenient site where the cells may find an appropriate site for regeneration and differentiation (*e*.*g*., thymus). For example, at least about 1 × 10⁵ cells can be administered, eventually reaching about 1 × 10¹⁰ or more, such as at least about 1 × 10⁶ cells. A cell population comprising immunoresponsive cells comprising a TCR can comprise a purified population of cells. Those skilled in the art can readily determine the percentage of immunoresponsive cells in a cell population using various well-known methods, such as fluorescence activated cell sorting (FACS). The ranges of purity in cell populations comprising genetically modified immunoresponsive cells comprising a TCR can be from about 50% to about 55%, from about 55% to about 60%, from about 65% to about 70%, from about 70% to about 75%, from about 75% to about 80%, from about 80% to about 85%; from about 85% to about 90%, from about 90% to about 95%, or from about 95 to about 100%. Dosages can be readily adjusted by those skilled in the art (e.g., a decrease in purity may require an increase in dosage). The immunoresponsive cells can be for administration by injection, catheter, or the like. If desired, factors can also be included, including, but not limited to, interleukins, e.g. IL-2, IL-3, IL 6, IL-11, IL-7, IL-12, IL-15, IL-21, as well as the other interleukins, the colony stimulating factors, such as G-, M- and GM-CSF, interferons, *e*.*g*., γ-interferon.

The immunoresponsive cells comprising a TCR and compositions comprising thereof can be obtained from one subject, and for administration to the same subject or a different, compatible subject. The peripheral blood derived T cells or their progeny (*e*.*g*., *in vivo, ex vivo or in vitro* derived) can be for administration via localized injection, including catheter administration, systemic injection, localized injection, intravenous injection, or parenteral administration. A pharmaceutical composition comprising immunoresponsive cells comprising a TCR can be formulated in a unit dosage injectable form (solution, suspension, emulsion). The compositions can further comprise a pharmaceutically acceptable carrier.

### VII. Formulations

Immunoresponsive cells comprising a TCR and compositions comprising thereof can be conveniently provided as sterile liquid preparations, *e*.*g*., isotonic aqueous solutions, suspensions, emulsions, dispersions, or viscous compositions, which may be buffered to a selected pH. Liquid preparations are normally easier to prepare than gels, other viscous compositions, and solid compositions. Additionally, liquid compositions are somewhat more convenient to administer, especially by injection. Viscous compositions, on the other hand, can be formulated within the appropriate viscosity range to provide longer contact periods with specific tissues. Liquid or viscous compositions can comprise carriers, which can be a solvent or dispersing medium containing, for example, water, saline, phosphate buffered saline, polyol (for example, glycerol, propylene glycol, liquid polyethylene glycol, and the like) and suitable mixtures thereof.

Sterile injectable solutions can be prepared by incorporating the compositions of the presently disclosed subject matter, *e.g.*, a composition comprising immunoresponsive cells comprising a presently disclosed TCR, in the required amount of the appropriate solvent with various amounts of the other ingredients, as desired. Such compositions may be in admixture with a suitable carrier, diluent, or excipient such as sterile water, physiological saline, glucose, dextrose, or the like. The compositions can also be lyophilized. The compositions can contain auxiliary substances such as wetting, dispersing, or emulsifying agents (*e*.*g*., methylcellulose), pH buffering agents, gelling or viscosity enhancing additives, preservatives, flavoring agents, colors, and the like, depending upon the route of administration and the preparation desired. Standard texts, such as "REMINGTON'S PHARMACEUTICAL SCIENCE", 17th edition, 1985 may be consulted to prepare suitable preparations.

Various additives which enhance the stability and sterility of the compositions, including antimicrobial preservatives, antioxidants, chelating agents, and buffers, can be added. Prevention of the action of microorganisms can be ensured by various antibacterial and antifungal agents, for example, parabens, chlorobutanol, phenol, sorbic acid, and the like. Prolonged absorption of the injectable pharmaceutical form can be brought about by the use of agents delaying absorption, for example, alum inurn monostearate and gelatin. However, any vehicle, diluent, or additive used would have to be compatible with the immunoresponsive cells comprising the TCR.

The compositions can be isotonic, *i*.*e*., they can have the same osmotic pressure as blood and lacrimal fluid. The desired isotonicity of the compositions of the presently disclosed subject matter may be accomplished using sodium chloride, or other pharmaceutically acceptable agents such as dextrose, boric acid, sodium tartrate, propylene glycol or other inorganic or organic solutes. Sodium chloride is preferred particularly for buffers containing sodium ions.

Viscosity of the compositions, if desired, can be maintained at the selected level using a pharmaceutically acceptable thickening agent. Methylcellulose can be used because it is readily and economically available and is easy to work with. Other suitable thickening agents include, for example, xanthan gum, carboxymethyl cellulose, hydroxypropyl cellulose, carbomer, and the like. The concentration of the thickener can depend upon the agent selected. The important point is to use an amount that will achieve the selected viscosity. Obviously, the choice of suitable carriers and other additives will depend on the exact route of administration and the nature of the particular dosage form, *e.g.*, liquid dosage form (*e*.*g*., whether the composition is to be formulated into a solution, a suspension, gel or another liquid form, such as a time release form or liquid-filled form).

Those skilled in the art will recognize that the components of the compositions should be selected to be chemically inert and will not affect the viability or efficacy of the immunoresponsive cells as describe in the presently disclosed subject matter. This will present no problem to those skilled in chemical and pharmaceutical principles, or problems can be readily avoided by reference to standard texts or by simple experiments (not involving undue experimentation), from this disclosure and the documents cited herein.

One consideration concerning the therapeutic use of the immunoresponsive cells is the quantity of cells necessary to achieve an optimal effect. The quantity of cells to be administered will vary for the subject being treated, for example from about 10⁴ to about 10¹⁰, from about 10⁵ to about 10⁹, or from about 10⁶ to about 10⁸ immunoresponsive cells of the presently disclosed subject matter are administered to a subject. More effective cells may be administered in even smaller numbers. In other examples, at least about 1 × 10⁸, about 2 × 10⁸, about 3 × 10⁸, about 4 × 10⁸, and about 5 × 10⁸ the immunoresponsive cells are to be administered to a human subject. The precise determination of what would be considered an effective dose may be based on factors individual to each subject, including their size, age, sex, weight, and condition of the particular subject. Dosages can be readily ascertained by those skilled in the art from this disclosure and the knowledge in the art.

The skilled artisan can readily determine the amount of cells and optional additives, vehicles, and/or carrier in compositions and to be administered in methods of the presently disclosed subject matter. Typically, any additives (in addition to the active cell(s) and/or agent(s)) are present in an amount of from about 0.001% to about 50% by weight) solution in phosphate buffered saline, and the active ingredient is present in the order of micrograms to milligrams, such as from about 0.0001 wt% to about 5 wt %, from about 0.0001 wt% to about 1 wt %, from about 0.0001 wt% to about 0.05 wt%, from about 0.001 wt% to about 20 wt %, from about 0.01 wt% to about 10 wt %, or from about 0.05 wt% to about 5 wt %. For any composition to be administered to an animal or human, and for any particular method of administration, toxicity should be determined, such as by determining the lethal dose (LD) and LD50 in a suitable animal model *e*.*g*., rodent such as mouse; and, the dosage of the composition(s), concentration of components therein and timing of administering the composition(s), which elicit a suitable response. Such determinations do not require undue experimentation from the knowledge of the skilled artisan, this disclosure and the documents cited herein. And, the time for sequential administrations can be ascertained without undue experimentation.

### VIII. Medical uses

Immunoresponsive cells comprising a TCR identified by the method of the present invention may be useful for treating a malignancy in a subject. The Immunoresponsive cells may be useful for administration in an amount effective to achieve the desired effect, be it palliation of an existing condition or prevention of recurrence. For treatment, the amount administered is an amount effective in producing the desired effect. An effective amount can be provided in one or a series of administrations. An effective amount can be provided in a bolus or by continuous perfusion.

For adoptive immunotherapy using antigen-specific T cells, cell doses in the range of about 10⁶ to about 10¹⁰ (*e*.*g*., about 10⁹ or about 10⁶) are typically infused. Upon administration of the immunoresponsive cells into the subject and subsequent differentiation, the immunoresponsive cells are induced that are specifically directed against one specific antigen (*e*.*g*., a tumor antigen). "Induction" of T cells can include inactivation of antigen-specific T cells such as by deletion or anergy. Inactivation is particularly useful to establish or reestablish tolerance such as in autoimmune disorders. The immunoresponsive cells can be for administration by any methods known in the art, including, but not limited to, pleural administration, intravenous administration, subcutaneous administration, intranodal administration, intratumoral administration, intrathecal administration, intrapleural administration, intraperitoneal administration, and direct administration to the thymus.

The immunoresponsive cells (*e*.*g*., T cells) comprising a TCR identified by the method of the present invention may for example be useful for reducing tumor burden in a subject by inducing tumor cell death in the subject. The immunoresponsive cell can reduce the number of tumor cells, reduce tumor size, and/or eradicate the tumor in the subject.

The immunoresponsive cells may be useful for increasing or lengthening survival of a subject having a neoplasm.

Cancers whose growth may be inhibited using the immunoresponsive cells of the presently disclosed subject matter comprise cancers typically responsive to immunotherapy. Examples of such cancers include solid malignancies, e.g., breast cancer, ovarian cancer, bladder cancer, pancreatic cancer and other solid malignancies.

Additionally, the immunoresponsive cells may be useful for increasing immune-activating cytokine production in response to a cancer cell in a subject. The immune-activating cytokine can be granulocyte macrophage colony stimulating factor (GM-CSF), IFN- α, IFN-β, IFN-γ, TNF-α, IL-2, IL-3, IL-6, IL-11, IL-7, IL-12, IL-15, IL-21, interferon regulatory factor 7 (IRF7), and combinations thereof. For example, the immunoresponsive cells including a TCR may increase the production of GM-CSF, IFN-γ, and/or TNF-α.

Suitable human subjects for therapy typically comprise two treatment groups that can be distinguished by clinical criteria. Subjects with "advanced disease" or "high tumor burden" are those who bear a clinically measurable tumor (*e*.*g*., breast cancer). A clinically measurable tumor is one that can be detected on the basis of tumor mass (*e*.*g*., by palpation, CAT scan, sonogram, mammogram or X-ray; positive biochemical or histopathologic markers on their own are insufficient to identify this population). A pharmaceutical composition comprising the immunoresponsive cells comprising the TCR identified by the method of the present invention may therefore be useful for eliciting an anti-tumor response, with the objective of palliating their condition. Ideally, reduction in tumor mass occurs as a result, but any clinical improvement constitutes a benefit. Clinical improvement comprises decreased risk or rate of progression or reduction in pathological consequences of the tumor (*e*.*g*., breast cancer).

A second group of suitable subjects is known in the art as the "adjuvant group." These are individuals who have had a history of neoplasm (*e*.*g*., breast cancer), but have been responsive to another mode of therapy. The prior therapy can have included, but is not restricted to, surgical resection, radiotherapy, and traditional chemotherapy. As a result, these individuals have no clinically measurable tumor. However, they are suspected of being at risk for progression of the disease, either near the original tumor site, or by metastases. This group can be further subdivided into high-risk and low-risk individuals. The subdivision is made on the basis of features observed before or after the initial treatment. These features are known in the clinical arts, and are suitably defined for each different neoplasm. Features typical of high-risk subgroups are those in which the tumor (*e*.*g*., breast cancer) has invaded neighboring tissues, or who show involvement of lymph nodes. Another group has a genetic predisposition to neoplasm (*e*.*g*., breast cancer) but has not yet evidenced clinical signs of neoplasm (*e*.*g*., breast cancer). For instance, women testing positive for a genetic mutation associated with breast cancer, but still of childbearing age, can wish to receive one or more of the TCRs described herein in treatment prophylactically to prevent the occurrence of neoplasm until it is suitable to perform preventive surgery.

The subjects can have an advanced form of disease (*e*.*g*., breast cancer), in which case the treatment objective can include mitigation or reversal of disease progression, and /or amelioration of side effects. The subjects can have a history of the condition, for which they have already been treated, in which case the therapeutic objective will typically include a decrease or delay in the risk of recurrence.

### IX. Kits

Disclosed herein are also kits for use in the treatment or prevention of a malignancy (*e*.*g*., breast cancer). Such kits may comprise a therapeutic or prophylactic composition containing an effective amount of an immunoresponsive cell comprising a TCR disclosed herein in unit dosage form. The cells further expresses at least one co-stimulatory ligand.

If desired, the immunoresponsive cell can be provided together with instructions for administering the cell to a subject having or at risk of developing a malignancy (*e*.*g*., breast cancer). The instructions will generally include information about the use of the composition for the treatment or prevention of a malignancy (*e*.*g*., breast cancer). In other embodiments, the instructions include at least one of the following: description of the therapeutic agent; dosage schedule and administration for treatment or prevention of a malignancy (*e*.*g*., breast cancer) or symptoms thereof; precautions; warnings; indications; counter-indications; over-dosage information; adverse reactions; animal pharmacology; clinical studies; and/or references. The instructions may be printed directly on the container (when present), or as a label applied to the container, or as a separate sheet, pamphlet, card, or folder supplied in or with the container.

### EXAMPLES

The practice of the present invention employs, unless otherwise indicated, conventional techniques of molecular biology (including recombinant techniques), microbiology, cell biology, biochemistry and immunology, which are well within the purview of the skilled artisan. Such techniques are explained fully in the literature, such as, "Molecular Cloning: A Laboratory Manual", second edition (Sambrook, 1989); "Oligonucleotide Synthesis" (Gait, 1984); "Animal Cell Culture" (Freshney, 1987); "Methods in Enzymology" "Handbook of Experimental Immunology" (Weir, 1996); "Gene Transfer Vectors for Mammalian Cells" (Miller and Calos, 1987); "Current Protocols in Molecular Biology" (Ausubel, 1987); "PCR: The Polymerase Chain Reaction", (Mullis, 1994); "Current Protocols in Immunology" (Coligan, 1991). These techniques are applicable to the production of the polynucleotides and polypeptides of the invention, and, as such, may be considered in making and practicing the invention. Particularly useful techniques for particular embodiments will be discussed in the sections that follow.

The following examples are put forth so as to provide those of ordinary skill in the art with a complete disclosure and description of how to make and use the compositions, and assay, and screening.

### Example 1: Identification of mutant PIK3CA-targeted TCRs

PIK3CA mutations are prevalent in many types of cancer. Therefore, there is a need to identify novel TCRs targeting specific mutations of PIK3CA for cancer immunotherapy.

To identify TCRs specifically targeting mutant PIK3CA, peripheral blood mononuclear cells (PBMCs) from healthy donors were stimulated with autologous mature antigen-presenting cells transfected with mRNA encoding a 65-amino acid segment of the PIK3CA gene flanking common hotspot mutations. The cells received *in vitro* antigen stimulation two/three times over a period of two/three weeks in the presence of low-dose IL-2 (90 IU/mL) (Figure 2). High throughput reactivity screen was then performed, where individual *in vitro* sensitized microwells were tested for mutation-specific reactivity by incubating with autologous APCs transfected with mRNA encoding either the wild-type (WT) or mutated PIK3CA (Figure 3). Mutation-specific recognition is determined by the preferential upregulation of mRNA transcript of acute inflammatory markers such as IFN-g and TNF-a by high throughput quantitative PCR (Figure 3). Mutation-specific T cells were then isolated, where positive microwells underwent limiting dilution cloning to derive T cell clones of a single specificity, and individual clones were screened for mutation-specific recognition using previously described methodology. Molecular sequencing was then performed on positive clones to derive the alpha and beta chain sequences of its T cell receptor (Figure 4). Further, mutant PIK3CA specific TCRs were reconstruction. The genetic sequences encoding the alpha and beta chains of the mutation-specific TCR were cloned into a retroviral vector and stably integrated into allogeneic PBMC (Figure 5). Conference of reactivity to mutation-specific transfectants and HLA-matched tumor cell line targets comprising the mutant antigen were determined by IFN-g secretion and upregulation of costimulatory molecules such as 4-1BB and OX40. A representative schema of this process is shown in Figure 5 as an example. Stimulation and screening constructs for mPIK3CA are shown in Figure 6.

Mutation-reactive microwells were identified following *in vitro* sensitization. As shown in Figure 7, delta CT values from sister wells against mutated or WT PI3KCA plotted on the X and Y axis respectively. Each dot represents an individual sensitized microwell. Mutation-specific recognition of the hotspot mutations, E542K in the top panel, E545K mutation in the middle panel and the H1047R/L in the bottom panel are also shown in Figure 7, where outliner dots identified by the arrows indicating wells that preferentially upregulate IFN-g transcript in response to mutated PIK3CA were selected to undergo limiting dilution cloning to derive mutation-specific T cell clones.

Clones derived from limiting dilution were further screened, where growth-positive wells following limiting dilution cloning were screened for PIK3CA mutation-specific recognition by measuring the preferential upregulation of the costimulatory molecule, OX40 (Figure 8, left panel) and/or the release of IFN-g (Figure 8, right panel) in response to the mutated antigen. Positive clones were selected to undergo molecular sequencing to derive the genetic code for the alpha and beta chains of their T cell receptor.

Similar screening was also performed with T cells derived from Donor 3 as show in Figure 9. Delta CT values from paired microwells against mutant or WT PIK3CA were plotted on the X and Y axis respectively. Each dot represented an individual sensitized microwell (n=384) sensitized against one of four PI3KCA hotspot mutations listed. Well C8 (bottom left) derived from a H1047R-sensitized IVS and Well B11 (bottom right) derived from a H1047L-sensitized IVS were mutation-specific, as determined by preferential upregulation of IFN-g mRNA to mutant antigen.

Single-cell sequencing was incorporated into the discovery platform, the benefit of which includes, but are not limited to: a) potential to remove requirement to perform limited dilution cloning and save at least 2 weeks of time; b) ensuring maximal TCR diversity is captured by avoiding limited dilution cloning, which minimizes risk of losing TCRs associated with slow or poor growing T cells; c) increased discovery throughput by reduction in staff labor to tend to micro-wells and cloning and by making certain steps amenable to automation; and d) increased accuracy in identifying reactive TCRs (if paired with full RNA-seq, can design screens to incorporate assessment of both TCR frequency with function). PBMCs were transduced with known TCRs (F5 = MART-1, 1G4 = NY-ESO-1). TCR transduced T cells were spiked at a known concentration into a bulk untransduced PBMC mixture. Single cell sequencing was used to assess whether the platform can: i) retrieve high quality and correctly paired TCR a/b sequences from the spiked samples, and ii) accurately quantify the frequency of the spiked samples (Figure 10). Figure 11 shows that sequencing correctly identified, paired, and quantified known TCRs within a bulk PBMC population.

Figure 12 shows the confirmation of RC8 reactivity. Autologous APCs transfected with RNA encoding either WT or the R/ L substitutions at position 1047 in the PIK3CA gene were incubated with T cells from Well C8 (referred to as RC8). Delta CT values determined by upregulation of IFN-g transcript indicated mutation-specific recognition of both R and L hotspot mutations and no WT recognition. The table in the lower panel of Figure 12 lists the CDR3 sequences and frequencies of the top 10 clonotypes in RC8 derived by the platform.

Figure 13 shows the confirmation of LB11 reactivity: Autologous APCs transfected with RNA encoding either WT or the R/L substitutions at position 1047 in the PIK3CA gene were incubated with T cells from Well B11 (referred to as LB11). Delta CT values determined by upregulation of IFN-g transcript indicated specific recognition of H1047L alone and no H1047R or WT recognition. The table in the lower panel of Figure 13 lists the CDR3 sequences and frequencies of the top 10 clonotypes in LB11 derived by the platform.

### Example 2: Modified vector to enhance in vitro T cell stimulation

Modified vectors were developed to enhance *in vitro* T cell stimulation, where one or more costimulatory ligands are expressed in the antigen presenting cells (APCs). Figure 14 shows that one or more costimulatory ligands, *e*.*g*., 4-1BBL and OX40L can be constructed in the same vector with an antigen of interest and be expressed in an APC cell to enhance T cell stimulation. The antigen and costimulatory ligands were joined by a 2A-peptide to enable polycistronic expression cassette. Codon-optimized P2A linker sequence (SEQ ID NO: 2) were used for improved gene expression in APCs. Alternative designs of the modified vectors are shown in Figure 15.

Costimulatory molecules were expressed on the surface after transient transfection with the modified vector, as shown in Figure 16. Monocyte derived dendritic cells were transfected with mRNA encoding a tumor antigen of interest and the costimulatory ligand 4-1BBL or OX40L expressed alone or in tandem. Histograms depicted the frequency of expression of 41BBL (top) and OX40L (bottom) at 24 hours post-transfection.

Co-electroporation of 4-1BBL and OX40L containing mini-genes did not compromise antigen expression, as shown in Figure 17. Monocyte derived dendritic cells were transfected with RNA encoding a tumor antigen as a model antigen of interest and the costimulatory ligand 41BBL or OX40L alone or in tandem. Bar graphs indicated frequency and mean fluorescence intensity (MFI) of the level of expression of 4-1BBL (top), OX40L (middle) and tumor antigen (bottom) at 24 hours post-transfection.

## Claims

1. An *in vitro* method for identifying a T cell receptor (TCR) that targets an antigen, comprising:
a) contacting a plurality of lymphocytes with a plurality of antigen presenting cells (APCs), wherein the APC comprises an antigen and at least one exogenous costimulatory ligand;
b) identifying a lymphocyte that is stimulated by the APC (APC-stimulated lymphocyte); and
c) identifying a TCR comprised by the APC-stimulated lymphocyte identified in b);
wherein the lymphocytes and the APCs are from a population of peripheral blood mononuclear cells of a single donor, and optionally wherein
(i) the APC is a mature APC,
(ii) the APC is a monocyte-derived dendritic cell; and/or
(iii) the lymphocyte is a T cell.

2. The method of claim 1, wherein the antigen
(i) is a tumor antigen or a pathogen antigen, optionally wherein the antigen is a small peptide derived from the tumor antigen; and/or
(ii) is selected from the group consisting of tumor associated antigens, cancer germline antigens, mutated/neoantigens and combinations thereof, optionally wherein the antigen is a small peptide derived from the tumor antigen; and/or
(iii) the antigen is a small peptide that comprises fewer than 24 amino acid residues; and/or
(iv) the antigen comprises a mutation.

3. The method of claim 1 or 2, wherein the at least one exogenous costimulatory ligand is selected from the group consisting of 4-1BBL, OX40L, CD40L, ICOSL, CD70, and combinations thereof.

4. The method of any one of claims 1-3, wherein the comprises two exogenous costimulatory ligands comprising 4-1BBL and OX40L.

5. The method of any one of claims 1-4, wherein the antigen and the costimulatory ligand are transfected into the APC.

6. The method of any one of claims 1-5, wherein the antigen and the costimulatory ligand are expressed through a single vector.

7. The method of any one of claims 1-6, wherein (i) the control lymphocyte is a lymphocyte that has not been contacted with an APC or a lymphocyte that has been contacted with an APC that does not comprise the antigen, and/or (ii) the cytokine is selected from the group consisting of IL-2, IFNγ, TNFα, MIP1α, granzyme B, perforin, CD107a, CD107b, TGF-β, IL-4, IL-3, IL-6, IL-7, IL-11, IL-12, IL-15, IL-17, IL-18, IL-21, and combinations thereof.

8. The method of claim 7, further comprising measuring the cytokine expression of the APC-stimulated lymphocyte and measuring the cytokine expression of the control lymphocyte; optionally wherein measuring the cytokine expression comprises polymerase chain reaction (PCR); further optionally wherein the PCR is a quantitative PCR (qPCR).

9. The method of any one of claims 1-8, wherein identifying the TCR comprises obtaining a sequence of the TCR, optionally wherein (i) the sequence comprises mRNA sequence, cDNA sequence, genomic DNA sequence, or a combination thereof; and/or (ii) obtaining the sequence of the TCR comprises nucleic acid sequencing.

10. The method of any one of claims 1-9, further comprising validating the binding specificity of the TCR to the antigen; optionally wherein validating the binding specificity of the TCR to the antigen comprises:
i) expressing the TCR in a lymphocyte,
ii) contacting the lymphocyte of step i) with an APC comprising the antigen, and
iii) validating the binding specificity of the TCR to the antigen when the lymphocyte of step ii) is stimulated by the APC, optionally wherein the stimulation of the lymphocyte by the APC is identified by detecting an increase of expression of a cytokine of the lymphocyte as compared to expression of the cytokine of a control lymphocyte, further optionally wherein
(a) the control lymphocyte is a lymphocyte that expresses the TCR and has not been contacted with an APC or a lymphocyte that expresses the TCR that has been contacted with an APC that does not comprise the antigen; and/or
(b) the cytokine is selected from the group consisting of IL-2, IFNγ, TNFα, MIP1α, granzyme B, perforin, CD107a, CD107b, TGF-β, IL-4, IL-3, IL-6, IL-7, IL-11, IL-12, IL-15, IL-17, IL-18, IL-21, and combinations thereof.

## Patentansprüche

1. In-vitro-Verfahren zum Identifizieren eines T-Zellrezeptors (TCR), der auf ein Antigen abzielt, umfassend:
a) Inkontaktbringen einer Vielzahl von Lymphozyten mit einer Vielzahl antigenpräsentierender Zellen (APCs), wobei die APC ein Antigen und mindestens einen exogenen kostimulatorischen Liganden umfasst;
b) Identifizieren eines Lymphozyten, der durch die APC stimuliert wird (APC-stimulierter Lymphozyt); und
c) Identifizieren eines TCR, der von dem APC-stimulierten Lymphozyten umfasst ist, der in b) identifiziert wurde; wobei die Lymphozyten und die APCs aus einer Population von mononukleären Zellen des peripheren Bluts eines einzelnen Spenders stammen und wobei optional
(i) die APC eine reife APC ist,
(ii) die APC eine von Monozyten abgeleitete dendritische Zelle ist; und/oder
(iii)der Lymphozyt eine T-Zelle ist.

2. Verfahren nach Anspruch 1, wobei das Antigen
(i) ein Tumorantigen oder ein Pathogenantigen ist, wobei das Antigen optional ein kleines Peptid ist, das von dem Tumorantigen abgeleitet ist; und/oder
(ii) aus der Gruppe ausgewählt ist, die aus tumorassoziierten Antigenen, Krebs-Keimbahn-Antigenen, mutierten Antigenen/Neoantigenen und Kombinationen davon besteht, wobei das Antigen optional ein kleines Peptid ist, das von dem Tumorantigen abgeleitet ist; und/oder
(iii) das Antigen ein kleines Peptid ist, das weniger als 24 Aminosäurereste umfasst; und/oder
(iv) das Antigen eine Mutation umfasst.

3. Verfahren nach Anspruch 1 oder 2, wobei der mindestens eine exogene kostimulatorische Ligand aus der Gruppe ausgewählt ist, die aus 4-1BBL, OX40L, CD40L, ICOSL, CD70 und Kombinationen davon besteht.

4. Verfahren nach einem der Ansprüche 1-3, wobei die zwei exogene kostimulatorische Liganden umfasst, die 4-1BBL und OX40L umfassen.

5. Verfahren nach einem der Ansprüche 1-4, wobei das Antigen und der kostimulatorische Ligand in die APC transfiziert werden.

6. Verfahren nach einem der Ansprüche 1-5, wobei das Antigen und der kostimulatorische Ligand durch einen einzelnen Vektor exprimiert werden.

7. Verfahren nach einem der Ansprüche 1-6, wobei (i) der Kontroll-Lymphozyt ein Lymphozyt ist, der nicht mit einer APC in Kontakt gebracht wurde, oder ein Lymphozyt ist, der mit einer APC in Kontakt gebracht wurde, die das Antigen nicht umfasst, und/oder (ii) das Zytokin aus der Gruppe ausgewählt ist, die aus IL-2, IFNγ, TNFα, MIP1α, Granzym B, Perforin, CD107a, CD107b, TGF-β, IL-4, IL-3, IL-6, IL-7, IL-11, IL-12, IL-15, IL-17, IL-18, IL-21 und Kombinationen davon besteht.

8. Verfahren nach Anspruch 7, ferner umfassend Messen der Zytokinexpression des APC-stimulierten Lymphozyten und Messen der Zytokinexpression des Kontroll-Lymphozyten; wobei optional Messen der Zytokinexpression eine Polymerasekettenreaktion (PCR) umfasst; wobei die PCR ferner optional eine quantitative PCR (qPCR) ist.

9. Verfahren nach einem der Ansprüche 1-8, wobei das Identifizieren des TCR Erhalten einer Sequenz des TCR umfasst, wobei optional (i) die Sequenz eine mRNA-Sequenz, cDNA-Sequenz, genomische DNA-Sequenz oder eine Kombination davon umfasst; und/oder (ii) Erhalten der Sequenz des TCR Nukleinsäuresequenzierung umfasst.

10. Verfahren nach einem der Ansprüche 1-9, ferner umfassend Validieren der Bindungsspezifität des TCR an das Antigen; wobei optional Validieren der Bindungsspezifität des TCR an das Antigen Folgendes umfasst:
i) Exprimieren des TCR in einem Lymphozyten,
ii) Inkontaktbringen des Lymphozyten aus Schritt i) mit einer APC, die das Antigen umfasst, und
iii) Validieren der Bindungsspezifität des TCR an das Antigen, wenn der Lymphozyt aus Schritt ii) durch die APC stimuliert wird, wobei optional die Stimulation des Lymphozyten durch die APC identifiziert wird, indem eine Erhöhung der Expression eines Zytokins des Lymphozyten im Vergleich zur Expression des Zytokins eines Kontroll-Lymphozyten nachgewiesen wird, wobei ferner optional
(a) der Kontroll-Lymphozyt ein Lymphozyt ist, der den TCR exprimiert und nicht mit einer APC in Kontakt gebracht wurde, oder ein Lymphozyt ist, der den TCR exprimiert, der mit einer APC in Kontakt gebracht wurde, die das Antigen nicht umfasst; und/oder
(b) das Zytokin aus der Gruppe ausgewählt ist, die aus IL-2, IFNγ, TNFα, MIP1α, Granzym B, Perforin, CD107a, CD107b, TGF-β, IL-4, IL-3, IL-6, IL-7, IL-11, IL-12, IL-15, IL-17, IL-18, IL-21 und Kombinationen davon besteht.

## Revendications

1. Procédé in vitro pour l'identification d'un récepteur de lymphocytes T (TCR) qui cible un antigène, comprenant :
a) la mise en contact d'une pluralité de lymphocytes avec une pluralité de cellules présentatrices d'antigène (APC), dans lequel l'APC comprend un antigène et au moins un ligand co-stimulateur exogène ;
b) l'identification d'un lymphocyte qui est stimulé par l'APC (lymphocyte stimulé par APC) ; et
c) l'identification d'un TCR constitué par le lymphocyte stimulé par APC identifié en b) ; dans lequel les lymphocytes et les APC proviennent d'une population de cellules mononucléées du sang périphérique d'un seul donneur, et éventuellement dans lequel
(i) l'APC est une APC mature,
(ii) l'APC est une cellule dendritique dérivée de monocytes ; et/ou
(iii) le lymphocyte est un lymphocyte T.

2. Procédé selon la revendication 1, dans lequel l'antigène
(i) est un antigène tumoral ou un antigène pathogène, éventuellement dans lequel l'antigène est un petit peptide dérivé de l'antigène tumoral ; et/ou
(ii) est choisi dans le groupe constitué d'antigènes associés à une tumeur, d'antigènes germinaux du cancer, d'antigènes/néo-antigènes mutés et de combinaisons de ceux-ci, éventuellement dans lequel l'antigène est un petit peptide dérivé de l'antigène tumoral ; et/ou
(iii) l'antigène est un petit peptide qui comprend moins de 24 résidus d'acides aminés ; et/ou
(iv) l'antigène comprend une mutation.

3. Procédé selon la revendication 1 ou 2, dans lequel l'au moins un ligand co-stimulateur exogène est choisi dans le groupe constitué de 4-1BBL, OX40L, CD40L, ICOSL, CD70 et de combinaisons de ceux-ci.

4. Procédé selon l'une quelconque des revendications 1 à 3, dans lequel comprend deux ligands co-stimulateurs exogènes comprenant 4-1BBL et OX40L.

5. Procédé selon l'une quelconque des revendications 1 à 4, dans lequel l'antigène et le ligand co-stimulateur sont transfectés dans l'APC.

6. Procédé selon l'une quelconque des revendications 1 à 5, dans lequel l'antigène et le ligand co-stimulateur sont exprimés par un seul vecteur.

7. Procédé selon l'une quelconque des revendications 1 à 6, dans lequel (i) le lymphocyte témoin est un lymphocyte qui n'a pas été mis en contact avec une APC ou un lymphocyte qui a été mis en contact avec une APC qui ne comprend pas l'antigène, et/ou (ii) la cytokine est choisie dans le groupe constitué de IL-2, IFNγ, TNFα, MIP1α, la granzyme B, la perforine, CD107a, CD107b, TGF-β, IL-4, IL-3, IL-6, IL-7, IL-11, IL-12, IL-15, IL-17, IL-18, IL-21 et des combinaisons de ceux-ci.

8. Procédé selon la revendication 7, comprenant en outre la mesure de l'expression de cytokine du lymphocyte stimulé par APC et la mesure de l'expression de cytokine du lymphocyte témoin ; dans lequel éventuellement, la mesure de l'expression de cytokine comprend une réaction en chaîne par polymérase (PCR) ; dans lequel en outre, éventuellement, la PCR est une PCR quantitative (qPCR).

9. Procédé selon l'une quelconque des revendications 1 à 8, dans lequel l'identification du TCR comprend l'obtention d'une séquence du TCR, dans lequel éventuellement (i) la séquence comprend une séquence d'ARNm, une séquence d'ADNc, une séquence d'ADN génomique ou une combinaison de celles-ci ; et/ou (ii) l'obtention de la séquence du TCR comprend le séquençage de l'acide nucléique.

10. Procédé selon l'une quelconque des revendications 1 à 9, comprenant en outre la validation de la spécificité de liaison du TCR à l'antigène ; dans lequel éventuellement, la validation de la spécificité de liaison du TCR à l'antigène comprend :
i) l'expression du TCR dans un lymphocyte,
ii) la mise en contact du lymphocyte de l'étape i) avec une APC comprenant l'antigène, et
iii) la validation de la spécificité de liaison du TCR à l'antigène lorsque le lymphocyte de l'étape ii) est stimulé par l'APC, dans lequel éventuellement la stimulation du lymphocyte par l'APC est identifiée en détectant une augmentation de l'expression d'une cytokine du lymphocyte par rapport à l'expression de la cytokine d'un lymphocyte témoin, dans lequel en outre éventuellement
(a) le lymphocyte témoin est un lymphocyte qui exprime le TCR et n'a pas été mis en contact avec une APC ou un lymphocyte qui exprime le TCR qui a été mis en contact avec une APC qui ne comprend pas l'antigène ; et/ou
(b) la cytokine est choisie dans le groupe constitué de IL-2, IFNγ, TNFα, MIP1α, la granzyme B, la perforine, CD107a, CD107b, TGF-β, IL-4, IL-3, IL-6, IL-7, IL-11, IL-12, IL-15, IL-17, IL-18, IL-21, et des combinaisons de ceux-ci.
